# EUROPÄISCHE PATENTANMELDUNG

(11) **EP 4 567 121 A1**
(43) Veröffentlichungstag der Anmeldung: **11.06.2025**
(21) Anmeldenummer: 23213897.4
(22) Anmeldetag: 04.12.2023
(51) Int. Cl.: C12P 7/10, A23D 9/02, A23K 10/00, A23L 33/18, C11B 1/10, C12M 1/00

(54) **KOMBINIERTES VERFAHREN ZUR HERSTELLUNG VON BIOETHANOL UND EINEM ÖLFRUCHTPROTEINPRODUKT**

(71) Anmelder: Agrana Beteiligungs- Aktiengesellschaft, 1020 Wien (AT)
(72) Erfinder: WASTYN, Marnik Michel, 2320 Schwechat (AT); PRANTER, Lukas, 3430 Tulln (AT); SCHUBERTH, Josef, 1020 Wien (AT); TEUFNER, Reinhard, 1020 Wien (AT)
(74) Vertreter: SONN Patentanwälte GmbH & Co KG

(57) **Zusammenfassung**

Die Erfindung betrifft ein kombiniertes Verfahren, umfassend ein erstes Verfahren zur Herstellung von Bioethanol (10) und ein zweites Verfahren zur Herstellung eines Ölfruchtproteinprodukts (31, 45), *das erste Verfahren umfassend die Schritte* (a) Bereitstellen einer Maische (7), wobei die Maische (7) ein kohlenhydrathaltiges Ausgangsmaterial (1), insbesondere ein Getreide, enthält, (b) Fermentieren der Maische (7), um eine fermentierte Brühe (9) enthaltend Bioethanol (10) zu erhalten, und (c) Abtrennen zumindest eines Teils des Bioethanols (10) von der fermentierten Brühe (9); und *das zweite Verfahren umfassend die Schritte* (i) Bereitstellen eines Ölfruchtausgangsprodukts (4), (ii) Extrahieren des Ölfruchtausgangsprodukts (4) mit einem ersten Extraktionsmittel (39), um eine erste Miscella (26) und ein erstes Extraktionsprodukt (27) zu erhalten, (iii) Trennen des ersten Extraktionsprodukts (27) in eine erste Mischung (30, 46) enthaltend das Ölfruchtproteinprodukt (31, 45) und in eine zweite Mischung (32, 47) enthaltend das Ölfruchtnebenprodukt (5), (iv) Abtrennen zumindest eines Teils des Ölfruchtproteinprodukts (31, 45) von der ersten Mischung (30, 46), und gegebenenfalls Abtrennen zumindest eines Teils des Ölfruchtnebenprodukts (5) von der zweiten Mischung (32, 47); wobei die in Schritt (a) des ersten Verfahrens bereitgestellte Maische (7) weiters zumindest einen Teil des im zweiten Verfahren erhaltenen Ölfruchtnebenprodukts (5) enthält, wobei das erste Extraktionsmittel (39) im ersten Verfahren hergestelltes Bioethanol (10) enthält, wobei die erste Miscella (26) zumindest einen Teil des ersten Extraktionsmittels (39) enthält, und wobei zumindest ein Teil des in der ersten Miscella (26) enthaltenen, ersten Extraktionsmittels (39) von der ersten Miscella (26) abgetrennt und in Schritt (c) des ersten Verfahrens verwendet wird.

## Beschreibung

Die Erfindung betrifft ein kombiniertes Verfahren zur Herstellung von Bioethanol und einem Ölfruchtproteinprodukt. Weiters betrifft die Erfindung eine kombinierte Anlage zur Herstellung des Bioethanols und des Ölfruchtproteinprodukts mit dem kombinierten Verfahren, sowie ein Futtermittel, das durch das kombinierte Verfahren erhältlich ist.

Der Einsatz von aus nachwachsenden Rohstoffen gewonnenem Bioethanol anstelle von aus Erdöl hergestelltem Ethanol hat in den letzten Jahren immer mehr an Bedeutung gewonnen. Ein Verfahren zur Herstellung von Ethanol aus Biomasse wird beispielsweise in der DE 10 2006 040 567 A1 beschrieben. Dabei wird die Biomasse zerkleinert und durch Fermentation wird daraus Bioethanol gewonnen. Demiray E. et al. (doi.org/10.1016/j.fuel.2020.119785) untersuchten die Auswirkung von löslichem Sojaprotein auf die enzymatische Hydrolyse von Apfeltrester für die Bioethanolproduktion. Sie kamen zu dem Ergebnis, dass lösliches Sojaprotein einen zweimal geringeren Enzymverbrauch und damit eine wirtschaftlichere enzymatische Hydrolyse und Bioethanolproduktion ermöglicht.

Auch Verfahren zur Verarbeitung von Ölfrüchten sind aus dem Stand der Technik bekannt, insbesondere Verfahren zur Verarbeitung von Sojabohnen zur Gewinnung von Sojaproteinprodukten. Beispielsweise lehrt die US 4,075,361 A ein Verfahren zur Herstellung eines essbaren Extrakts aus einer Ölsaat, wobei die Ölsaat in Gegenwart einer heißen alkalischen Extraktionslösung, die vorzugsweise Natriumhydroxid enthält, zerkleinert wird. Nach dem Extraktionsschritt wird die Mischung aus zerkleinerter Ölsaat und Extraktionslösung mit einer sauren Lösung neutralisiert und anschließend zentrifugiert.

Die US 4,457,869 A offenbart ein Verfahren zur Extraktion einer Ölsaat, bei dem die Ölsaat mit einem Lösungsmittel auf Isopropanolbasis in einem Extraktor in Kontakt gebracht wird, um eine flüssige Miscella und ein Mehl zu erhalten. Durch Kühlen und Phasentrennung wird Lösungsmittel von der Miscella abgetrennt und rückgewonnen, und durch Verdampfen wird Lösungsmittel vom Mehl abgetrennt und rückgewonnen.

Die EP 3 295 803 A1 offenbart ein Verfahren zur Gewinnung von Proteinpräparaten aus Sonnenblumensamen. Dabei werden die Sonnenblumenschalen geschält und anschließend durch Pressen teilentölt. Danach werden Extraktionsschritte mit einem Lösungsmittel durchgeführt, wodurch ein entfettetes, proteinhaltiges Mehl als Proteinpräparat erhalten wird. Eine Kombination von zwei oder mehr Extraktionslösungsmittel unterschiedlicher Polarität kann zum Einsatz kommen, z.B. Wasser, Säuren, Alkohole (z.B. Isopropanol, Ethanol, Methanol), Ketone (z.B. Aceton), Ether, Alkane (n-Hexan-, iso-Hexan). Dabei kann zuerst eine lipophile Extraktion durchgeführt werden und danach eine hydrophile Extraktion, oder umgekehrt.

Die WO 2022/006165 A1 und die WO 2022/183031 A1 beziehen sich auf Vorrichtungen, Systeme und Verfahren zur Verarbeitung von Sojabohnen mit einem alkoholbasierten Lösungsmittel, um Öl zu extrahieren. Bei dem alkoholbasierten Lösungsmittel kann es sich um Ethanol (z.B. Ethanol aus erneuerbaren Rohstoffen, einschließlich aus Rohstoffen aus biologischem Anbau) oder Isopropylalkohol handeln.

Die DE 27 52 595 A1 betrifft ein Verfahren zur Herstellung eines Proteinkonzentrats. Dabei wird ein ölhaltiges Samenmaterial (z.B. Sojabohnen) in vier aufeinanderfolgenden Stufen mit wässrigalkoholischen Lösungen (z.B. Lösungen von Alkohol in Wasser) extrahiert.

Die WO 2022/043248 A1 bezieht sich auf ein Verfahren zur industriellen Gewinnung von kaltgepresstem Kernöl, wobei Körner einer ölhaltigen Saat (z.B. Soja) geschält und von Schalen von einer schalenarmen Kornfraktion abgetrennt werden, und das kaltgepresste Kernöl von der schalenarmen Kornfraktion abgepresst wird. Eine Teilmenge des entstehenden Presskuchens wird zurückgeführt, mit der schalenarmen Kornfraktion vermengt und erneut abgepresst. Der verbleibende Presskuchen wird mit einem Lösungsmittel (z.B. Hexan oder Ethanol) extrahiert und zu einem Proteinkonzentrat getrocknet.

Bei der Herstellung von Ölfruchtproteinprodukten, insbesondere Ölfruchtproteinkonzentrat oder Ölfruchtproteinisolat, aus Ölfrüchten fällt ein Ölfruchtnebenprodukt an, das insbesondere Ölfruchtmelasse und/oder Ölfruchtproteinliquor enthalten kann. Dieses Ölfruchtnebenprodukt lässt sich zwar als Zusatz in Tierfutter verwenden, wird aber meist verbrannt, weil die anfallenden Mengen für eine komplette Verwertung für Fütterungszwecke zu groß sind. Es besteht ein Bedarf an einem Verfahren, das die Verwertung einer größeren Menge des bei der Herstellung von Ölfruchtproteinprodukten anfallenden Ölfruchtnebenprodukts ermöglicht und so die Nachhaltigkeit des Verfahrens erhöht. Die Nutzung des Abfallproduktes aus der Ölfruchtproteinproduktion als Rohstoff für die Herstellung von Bioethanol ermöglicht die Produktion von Bioethanol zweiter Generation. Andererseits ist es auch bei Herstellungsverfahren von Bioethanol wünschenswert, diese optimal zu gestalten - sowohl hinsichtlich deren Energiebilanz und CO₂-Fußabdruck, jedoch auch hinsichtlich der Nutzung der dabei hergestellten Produkte oder Zwischenprodukte. Insbesondere wünschenswert sind dabei auch Verfahren oder Kombinationen von Verfahren, die einen Produktions- oder Energiekreislauf im Sinne einer Bioraffinerie unterstützen oder ermöglichen.

Demgemäß betrifft die Erfindung ein kombiniertes Verfahren, umfassend ein erstes Verfahren zur Herstellung von Bioethanol und ein zweites Verfahren zur Herstellung eines Ölfruchtproteinprodukts,
*das erste Verfahren umfassend die Schritte*
   (a) Bereitstellen einer Maische, wobei die Maische ein kohlenhydrathaltiges Ausgangsmaterial, insbesondere ein Getreide, enthält,
   (b) Fermentieren der Maische, um eine fermentierte Brühe enthaltend Bioethanol zu erhalten, und
   (c) Abtrennen zumindest eines Teils des Bioethanols von der fermentierten Brühe; und
*das zweite Verfahren umfassend die Schritte*
   (i) Bereitstellen eines Ölfruchtausgangsprodukts,
   (ii) Extrahieren des Ölfruchtausgangsprodukts mit einem ersten Extraktionsmittel, um eine erste Miscella und ein erstes Extraktionsprodukt zu erhalten,
   (iii) Trennen des ersten Extraktionsprodukts in eine erste Mischung enthaltend das Ölfruchtproteinprodukt und in eine zweite Mischung enthaltend das Ölfruchtnebenprodukt,
   (iv) Abtrennen zumindest eines Teils des Ölfruchtproteinprodukts von der ersten Mischung, und gegebenenfalls Abtrennen zumindest eines Teils des Ölfruchtnebenprodukts von der zweiten Mischung;

   wobei die in Schritt (a) des ersten Verfahrens bereitgestellte Maische weiters zumindest einen Teil des im zweiten Verfahren erhaltenen Ölfruchtnebenprodukts enthält oder zumindest ein Teil des im zweiten Verfahren erhaltenen Ölfruchtnebenprodukts während Schritt (b) zugesetzt wird,
   wobei das erste Extraktionsmittel im ersten Verfahren hergestelltes Bioethanol enthält,
      wobei die erste Miscella zumindest einen Teil des ersten Extraktionsmittels enthält, und
      wobei zumindest ein Teil des in der ersten Miscella enthaltenen, ersten Extraktionsmittels von der ersten Miscella abgetrennt und in Schritt (c) des ersten Verfahrens verwendet wird.

Die vorliegende Erfindung betrifft daher die interaktive Kombination zweier an sich völlig unabhängiger Verfahren, wodurch sich überraschenderweise viele Synergien nutzen lassen. Dabei können die beiden Verfahren relativ flexibel gestaltet werden, insbesondere auf Basis der aktuellsten Ausführungsformen, da die gezielte Vernetzung der beiden Verfahren, wie sie erfindungsgemäß erzielt wird, an den Punkten vorgenommen werden, die den derzeit gängigen Einzelverfahren entsprechen. So kann das erste Verfahren gemäß etablierter Prozessschritten durchgeführt werden (wie sie z.B. in der DE 10 2006 040 567 A1 und vielen anderen Dokumenten beschrieben werden). Das zweite Verfahren kann im Wesentlichen ebenfalls gemäß gängiger Verfahren durchgeführt werden, soweit die Extraktion mit Ethanol als Extraktionsmittel durchgeführt wird, wodurch die erfinderische Kombination zwischen erstem und zweitem Verfahren begründet wird (s. z.B. WO 2022/006165 A1, WO 2022/183031 A1, WO 2022/043248 A1).

Erfindungsgemäß wird das im zweiten Verfahren anfallende Ölfruchtnebenprodukt vorzugsweise für die im ersten Verfahren bereitgestellte Maische verwendet und zusammen mit dem kohlenhydrathaltigen Ausgangsmaterial fermentiert. Dadurch kann nicht nur das Ölfruchtnebenprodukt einer Weiterverwendung zugeführt werden, sondern kann auch die Menge des im ersten Verfahren gewonnenen Bioethanols gesteigert werden. Des Weiteren bietet die Kombination dieser beiden Verfahren überraschende energetische und prozesstechnische Vorteile. Beispielsweise kann die im kombinierten Verfahren gewonnene thermische Energie auch im kombinierten Verfahren genutzt werden, wodurch die Verfahrenseffizienz maßgeblich verbessert werden kann. Vor allem ermöglicht es die Kombination der beiden Verfahren, im ersten Verfahren hergestelltes Bioethanol im zweiten Verfahren als erstes Extraktionsmittel zu verwenden und nachfolgend zur Reinigung in das erste Verfahren rückzuführen, wodurch eine Entwässerung des ersten Extraktionsmittels entfallen kann. Außerdem können, wenn das im zweiten Verfahren bereitgestellte Ölfruchtausgangsprodukt geschälte Ölfrüchte enthält, von den Ölfrüchten abgetrennte Schalen im ersten Verfahren genutzt werden und in der bereitgestellten Maische enthalten sein. Da die Schalen eine abrasive Wirkung haben, können sie die Verschmutzung von Anlagenkomponenten verringern, was sich positiv auf die Lebensdauer der Anlagen auswirkt. Auch geht mit dem Zusatz der Schalen zur Maische eine Aufwertung der Schalen einher, da die Schalen am Markt ansonsten nur schwer absetzbar sind.

### Erstes Verfahren - Herstellung des Bioethanols

In Schritt (a) des ersten Verfahrens wird die Maische bereitgestellt, die das kohlenhydrathaltige Ausgangsmaterial und vorzugsweise zumindest einen Teil des im zweiten Verfahren erhaltenen Ölfruchtnebenprodukts enthält. Unter einem kohlenhydrathaltigen Ausgangsmaterial wird ein Ausgangsmaterial verstanden, das ein oder mehrere Kohlenhydrat(e) enthält, beispielsweise ein Getreide, Kartoffeln, Hülsenfrüchte und/oder Rüben. Das kohlenhydrathaltige Ausgangsmaterial ist vorzugsweise ein Zuckerprodukt (z.B. Zuckerrübe, Zuckerhirse, Zuckerrohr, Früchte) oder ein Stärkeprodukt, wobei das Stärkeprodukt bevorzugt ein Getreide (z.B. Reis, Weizen, Mais, Roggen, Gerste), Kartoffeln und/oder Hülsenfrüchte (z.B. Bohnen, Linsen, Erbsen, Kichererbsen) enthält. Unter einem Stärkeprodukt wird gemäß der vorliegenden Erfindung ein kohlenhydrathaltiges Ausgangsmaterial verstanden, das eine oder mehrere Stärke(n) enthält. Vorzugsweise ist das kohlenhydrathaltige Ausgangsmaterial ein Getreide, besonders bevorzugt Mais und/oder Weizen. Hieraus kann eine vergleichsweise hohe Ausbeute an Bioethanol erhalten werden, insbesondere wenn ein enzymatischer Abbau vorgenommen wird und/oder wenn die Materialien (die enthaltenen Fasern) vorher vermahlen werden.

Das kohlenhydrathaltige Ausgangsmaterial und/oder das Ölfruchtnebenprodukt (wenn vorhanden) liegen in der Maische zumindest teilweise in einem gemaischten Zustand vor. Unter dem gemaischten Zustand wird ein Zustand verstanden, in welchem das kohlenhydrathaltige Ausgangsmaterial bzw. das Ölfruchtnebenprodukt ein zumindest teilweise oder zur Gänze aufgeschlossenes (d.h. verzuckertes) und somit fermentierbar gemachtes Kohlenhydrat (insbesondere eine Stärke), oder eine Mischung von zwei oder mehr solcher Kohlenhydrate, enthält.

Die in Schritt (a) bereitgestellte Maische kann durch Maischen des kohlenhydrathaltigen Ausgangsmaterials und gegebenenfalls des Ölfruchtnebenprodukts, vorzugsweise in Anwesenheit von Wasser, erhalten werden. Durch das Maischen kann ein im kohlenhydrathaltigen Ausgangsmaterial und/oder im Ölfruchtnebenprodukt enthaltenes Kohlenhydrat, insbesondere eine Stärke, aufgeschlossen und fermentierbar gemacht werden. Das Maischen wird vorzugsweise bei einer Temperatur von 60 bis 130 °C durchgeführt, bevorzugter bei 80 bis 120 °C. Dann ist nicht nur ein effizientes Maischen möglich, sondern kann auch eine thermische Schädigung des kohlenhydrathaltigen Ausgangsmaterials und des Ölfruchtnebenprodukts vermieden werden. Vorzugsweise erfolgt das Maischen für eine Zeitdauer von 0,5 h oder darüber, bevorzugter für 2 h oder darüber. Dann kann das Kohlenhydrat, insbesondere die Stärke, effizient aufgeschlossen werden. Besonders bevorzugt erfolgt das Maischen für eine Zeitdauer von 1 bis 4 h. Vor dem Maischen kann eine Saccharifizierung (Verzuckerung) vorgenommen werden, bei der längerkettige Kohlenhydrate in einzelne (oder zumindest kurzkettigere) Zuckermoleküle umgewandelt werden, wobei die Monosaccharidmoleküle intakt bleiben, als z.B. die Spaltung der Stärke in Glukose-Monomere. Das Maischen kann aber auch gleichzeitig oder überlappend mit der Verzuckerung vorgenommen werden. Daraus kann eine gute Balance zwischen Verfahrenseffizienz und Verzuckerung resultieren.

Das Maischen kann in Anwesenheit eines Enzyms oder mehreren Enzymen erfolgen. Das Enzym ist vorzugsweise ausgewählt aus der Gruppe umfassend eine Alpha-Amylase, eine Glucanase, eine Glucoamylase, eine Cellulase, eine Hemicellulase, eine Beta-Glucanase, eine Xylanase, eine Arabinase, eine Galactosidase und eine Protease, oder eine Mischung davon, wobei das Enzym besonders bevorzugt eine Alpha-Amylase und/oder eine Glucoamylase ist. Dann kann das Kohlenhydrat, insbesondere die Stärke, effizient aufgeschlossen werden.

Vor dem Maischen kann das kohlenhydrathaltige Ausgangsmaterial zerkleinert, insbesondere gemahlen, werden, vorzugsweise mittels Trockenmahlen und/oder Nassmahlen. Unter dem Trockenmahlen wird eine Zerkleinerung des kohlenhydrathaltigen Ausgangsmaterials im trockenen Zustand verstanden, d.h. ohne den Zusatz von Wasser. Beim Nassmahlen wird das kohlenhydrathaltige Ausgangsmaterial hingegen in Gegenwart einer vorgehend zugegebenen Flüssigkeit, insbesondere Wasser, zerkleinert. Das Ölfruchtnebenprodukt kann dem kohlenhydrathaltigen Ausgangsmaterial nach dessen Zerkleinern und vor dem Maischen zugegeben werden.

Beim Zerkleinern, insbesondere beim Trockenmahlen, werden das kohlenhydrathaltige Ausgangsmaterial und Schalen von Ölfrüchten vorzugsweise gemeinsam zerkleinert, wobei die Schalen der Ölfrüchte besonders bevorzugt zumindest ein Teil von in Schritt (i) des zweiten Verfahrens von Ölfrüchten, insbesondere Sojabohnen, abgetrennten Schalen sind. Dies ermöglicht erfindungsgemäß eine höherwertige Verwendung der Schalen der Ölfrüchte, die ansonsten oft verbrannt werden, zu Einzelfuttermittel, wie "Dried Destillers Grains with Solubles" ("DDGS"), welches als Nebenerzeugnis bei der Bioethanolherstellung aus Getreide gewonnen wird, wobei die anfallenden Destillationsrückstände getrocknet werden.

Beim Zerkleinern, insbesondere beim Nassmahlen, können Fasern, Maiskleber und/oder Maiskleberfutter (corn gluten feed, CGF) anfallen. Zumindest ein Teil davon kann vor dem Maischen vom zerkleinerten, kohlenhydrathaltigen Ausgangsmaterial abgetrennt werden. Die abgetrennten Fasern, der Maiskleber und/oder das Maiskleberfutter können einer von der fermentierten Brühe abgetrennten Schlempe (d.h. einem nicht fermentierbaren Rückstand) zugegeben werden. Wird die Schlempe als Futtermittel verwendet, können die Fasern, der Maiskleber und/oder das Maiskleberfutter als hochwertiger Futtermittelzusatz dienen. Maiskleber ist ein Proteinkonzentrat, das aus der Gewinnung von Maisstärke anfällt. CGF ist die Faserfraktion, die aus der Maisstärkegewinnung anfällt, wobei diese Fasern mit CSL (Corn Steep Liquor) gemeinsam getrocknet werden, wodurch CGF entsteht. CSL entsteht bei der Quellung des Mais im Vorfeld der Maisstärkegewinnungsprozess. Üblicherweise wird Bioethanol aus gemahlenem Mais und/oder Weizenmehl hergestellt. Es gibt aber auch Prozesse, wo z.B. Mais zuerst zu Stärke raffiniert wird und erst die Stärke für die Herstellung von Bioethanol herangezogen wird. Bioethanol aus nassvermahlenem Mais ist z.B. in den USA die dominierende Herstellungsvariante.

Die in Schritt (a) des ersten Verfahrens bereitgestellte Maische enthält vorzugsweise zumindest einen Teil des Ölfruchtnebenprodukts des zweiten Verfahrens. Die Maische kann insbesondere zumindest einen Teil der in Schritt (iii) erhaltenen zweiten Mischung (die das Ölfruchtnebenprodukt enthält) und/oder zumindest einen Teil des in Schritt (iv) erhaltenen Ölfruchtnebenprodukts (nach dessen Abtrennung von der zweiten Mischung, und gegebenenfalls nach einer Eindampfung) enthalten. Das Ölfruchtnebenprodukt kann eine Ölfruchtmelasse und/oder einen Ölfruchtproteinliquor enthalten, insbesondere eine Sojamelasse und/oder einen Sojaproteinliquor; dies kann von der Ausgestaltung des zweiten Verfahrens abhängen. Wenn im zweiten Verfahren ein Ölfruchtproteinkonzentrat hergestellt wird, kann die Ölfruchtmelasse im Ölfruchtnebenprodukt enthalten sein. Wird hingegen ein Ölfruchtproteinisolat hergestellt, kann der Ölfruchtproteinliquor im Ölfruchtnebenprodukt enthalten sein. Vorzugsweise weist das Ölfruchtnebenprodukt ein Trockensubstanzgewicht von 10 bis 80 Gew% auf, bevorzugter von 15 bis 50 Gew%, noch bevorzugter von 20 bis 40 Gew%, besonders bevorzugt von 25 bis 35 Gew%, bezogen auf das Gesamtgewicht des Ölfruchtnebenprodukts. Dies kann die Verfahrensführung beim Maischen und Fermentieren erleichtern. Unter diesem Trockensubstanzgewicht wird das Trockensubstanzgewicht verstanden, welches das Ölfruchtnebenprodukt unmittelbar vor seiner Verwendung im ersten Verfahren hat, gegebenenfalls nach einer Eindampfung.

Vorzugsweise enthält die in Schritt (a) bereitgestellte Maische das Ölfruchtnebenprodukt in einer Menge von 30 Gew% oder darunter, bevorzugter 20 Gew% oder darunter, noch bevorzugter 15 Gew% oder darunter, noch bevorzugter 12 Gew% oder darunter, noch bevorzugter 10 Gew% oder darunter, bezogen auf das Gewicht des kohlenhydrathaltigen Ausgangsmaterials. Bei Überschreiten dieser Menge an Ölfruchtnebenprodukt kann es zu einer Verschlechterung der Fermentationseigenschaften der Maische kommen. Bevorzugt ist es, wenn die in Schritt (a) bereitgestellte Maische das Ölfruchtnebenprodukt in einer Menge von 1 bis 30 Gew% enthält, vorzugsweise 1 bis 20 Gew%, bevorzugter 1 bis 15 Gew%, noch bevorzugter 1 bis 12 Gew%, besonders bevorzugt 5 bis 10 Gew%, bezogen auf das Gewicht des in der Maische enthaltenen kohlenhydrathaltigen Ausgangsmaterials. Dann kann eine gute Fermentationskinetik erreicht werden, was die Verfahrenseffizienz verbessert. Die Fermentationskinetik kann insbesondere dann optimal sein, wenn das kohlenhydrathaltige Ausgangsmaterial ein Getreide ist. Bevorzugt weist die Maische ein Verhältnis von Ölfruchtnebenprodukt zu kohlenhydrathaltigem Ausgangsmaterial, insbesondere Getreide, von 0,1:10 bis 1,5:10 auf, besonders bevorzugt von 1:10 (Gew%/Gew%). Hieraus resultiert ein besonders gutes Fermentationsverhalten, d.h. sowohl das kohlenhydrathaltige Ausgangsmaterial als auch das Ölfruchtnebenprodukt können gut fermentiert werden.

Bevorzugt ist es, wenn die in Schritt (a) bereitgestellte Maische Schalen von Ölfrüchten enthält, insbesondere zerkleinerte Schalen von Ölfrüchten. Diese Schalen sind vorzugsweise zumindest ein Teil von in Schritt (i) des zweiten Verfahrens von Ölfrüchten, insbesondere Sojabohnen, abgetrennten Schalen. Da die Schalen eine abrasive Wirkung haben, können sie die Verschmutzung von Anlagenkomponenten verringern, was sich positiv auf die Lebensdauer der Anlagen auswirkt. Auch geht mit dem Zusatz der Schalen zur Maische eine Aufwertung der Schalen einher, da die Schalen am Markt ansonsten nur schwer absetzbar sind. Die Schalen sind nicht bzw. nur teilweise fermentierbar und können in der von der fermentierten Brühe abgetrennten Schlempe enthalten sein. Bei Verwendung der Schlempe als Futtermittel kann es aufgrund der Schalen zwar zur Reduzierung des Proteingehalts des Futtermittels kommen. Jedoch hat die Anwesenheit der Schalen (insbesondere der zerkleinerten Schalen) in der Schlempe prozesstechnische Vorteile, denn dann kann das Trocknen der Schlempe effizienter erfolgen, da die (zerkleinerten) Schalen Wasser gut absorbieren können, insbesondere dann, wenn sie faserig sind, wobei sich faserige Schalen insbesondere durch Zerkleinern der Schalen ausbilden können.

In Schritt (b) des ersten Verfahrens wird die Maische fermentiert (d.h. vergärt). Dadurch wird Zucker (z.B. Maltose, Glucose) in Bioethanol umgewandelt. Kohlenstoffdioxid (CO₂) kann dabei als Nebenprodukt anfallen. Vorzugsweise wird der Maische vor und/oder in Schritt (b) ein Mikroorganismus zugegeben, insbesondere eine Hefe (wie z.B. Saccharomyces cerevisiae, Saccharomyces pastorianus) und/oder ein Bakterium (z.B. Zymomonas mobilis). Dann kann das Fermentieren besonders effizient erfolgen. Vorzugsweise wird die Maische bei einer Temperatur von 20 bis 40 °C fermentiert, bevorzugter von 28 bis 36 °C. Das Fermentieren kann dann effizient und ohne eine thermische Schädigung erfolgen. Vorzugsweise wird die Maische für eine Zeitdauer von 25 h oder darüber fermentiert, bevorzugter für 55 h oder darüber, besonders bevorzugt für eine Zeitdauer von 50 bis 70 h. Daraus kann eine gute Bioethanolausbeute bei zugleich guter Verfahrenseffizienz resultieren. Durch das Fermentieren wird eine fermentierte Brühe erhalten, die Bioethanol sowie eine Schlempe enthält. Unter der Schlempe wird der nicht fermentierbare Rückstand verstanden, und darin sind die Substanzen enthalten, die auch in der Maische enthalten waren. Die Schlempe enthält vorzugsweise ein Protein, Biomasse des Mikroorganismus, ein Fett und/oder einen Mineralstoff (bzw. eine Mischung von Proteinen, Fetten und/oder Mineralstoffen), weiters enthält die Schlempe vorzugweise Wasser. Nicht fermentierbare Kohlenhydrate können in der Schlempe enthalten sein, zumindest ein Großteil davon wurde durch Maischen verzuckert und dadurch fermentierbar gemacht.

In Schritt (c) des ersten Verfahrens wird zumindest ein Teil des Bioethanols von der fermentierten Brühe abgetrennt. Vorzugsweise wird in Schritt (c) auch zumindest ein Teil der in der fermentierten Brühe enthaltenen Schlempe von der fermentierten Brühe abgetrennt. Das Abtrennen des Bioethanols und/oder der Schlempe umfasst vorzugsweise ein thermisches Abtrennen, insbesondere ein Destillieren. Da Bioethanol bei einer niedrigeren Temperatur verdampft als Wasser (das ebenfalls in der fermentierten Brühe enthalten ist), kann der Bioethanol durch das thermische Abtrennen, insbesondere Destillieren, nicht nur von der in der fermentierten Brühe enthaltenen Feststoffen (die in der Schlempe enthalten sind), sondern auch zumindest von einem Teil des in der fermentierten Brühe enthaltenen Wassers abgetrennt werden. Durch das thermische Abtrennen, insbesondere Destillieren, kann die Schlempe von einer Bioethanol-Wasser-Mischung abgetrennt werden. Die Bioethanol-Wasser-Mischung enthält vorzugsweise einen Anteil von Bioethanol von 50 Gew% oder darüber, bevorzugter 85 Gew% oder darüber, noch bevorzugter nahe dem Azeotrop (95,6 Gew%), also 90 bis 95,6 Gew% bezogen auf das Gewicht der Bioethanol-Wasser-Mischung.

Das Abtrennen zumindest eines Teils des Bioethanols von der fermentierten Brühe in Schritt (c) umfasst bevorzugt das Destillieren und ein nachfolgendes Trennen der Bioethanol-Wasser-Mischung in Bioethanol und Wasser. Dadurch ist eine Entwässerung des Bioethanols möglich. Das Trennen der Bioethanol-Wasser-Mischung in Bioethanol und Wasser kann mittels eines Molekularsiebs, eines Membranverfahrens (unter Verwendung einer Membran) und/oder einer Druckwechseladsorption (unter Verwendung eines Adsorbens) erfolgen. Vorzugsweise erfolgt das Trennen der Bioethanol-Wasser-Mischung in Bioethanol und Wasser mittels eines Molekularsiebs. Hierdurch kann das Bioethanol effizient entwässert werden. Die Temperatur beim Trennen mittels des Molekularsiebs ist in der Regel abhängig vom Hersteller, liegt aber vorzugsweise im Bereich von 120 bis 180 °C, bevorzugter von 160 bis 170 °C, und/oder der Druck liegt vorzugsweise im Bereich von 2 bis 6 bar, bevorzugter von 3 bis 5 bar. Vorzugsweise weist das Molekularsieb eine Porenweite von 0,25 bis 0,35 nm auf, insbesondere 0,30 nm. Durch das Trennen der Bioethanol-Wasser-Mischung, insbesondere mittels des Molekularsiebs, kann im ersten Verfahren Bioethanol mit einer Reinheit von 99,0 Gew% oder darüber erhalten werden, vorzugsweise von 99,5 Gew% oder darüber, besonders bevorzugt von 99,9 Gew% oder darüber. Auch kann das Trennen aufgrund der Regenerierbarkeit und Wiederverwendbarkeit des Molekularsiebes nachhaltig erfolgen. Das vom Bioethanol abgetrennte Wasser wird vorzugsweise aufbereitet (d.h. gereinigt), sodass es wiederverwendet werden kann.

Die Entwässerung des Molekularsiebs, um dieses zu regenerieren, erfolgt vorzugsweise mit einem Reinigungs- bzw. Entwässerungsmittel. Dadurch können organische Rückstände entfernt werden. Diese Entwässerung (Regenerierung) kann in regelmäßigen Abständen erfolgen, bzw. je nach Bedarf. Vorzugsweise erfolgt die Entwässerung des Molekularsiebs mit einem ethanolhaltigen Reinigungsmittel (vorzugsweise als Regenerat mit dem Ethanol aus dem vorliegenden Verfahren), wobei das Reinigungsmittel vorzugsweise Ethanol in einer Menge von 95 Gew% oder darüber enthält, bevorzugter 97 Gew% oder darüber, noch bevorzugter 99,9 Gew% oder darüber, besonders bevorzugt 100 Gew%, bezogen auf das Gewicht des Reinigungsmittels. Besonders bevorzugt ist erfindungsgemäß die Verwendung des Ethanols, welches im ersten Verfahren hergestellt wurde, als Regenerat zur Entwässerung des Molekularsiebs. Dieses Regenerat kann dabei direkt (zumindest) für die zweite Extraktionsstufe verwendet werden, da es in der Regel einen Ethanolgehalt von über 80 Gew% aufweist und daher entweder direkt oder nach Verdünnung (vorzugsweise mit Wasser) verwendet werden kann. Diese Ausführungsform ist energetisch besonders vorteilhaft, da damit eine (Vor-)Destillation des Entwässerungsmittel nicht erforderlich ist.

Zur Reinigung des Molekularsiebs können ca. 0,1 bis 0,2 m³ Ethanol pro m³ an im ersten Verfahren hergestelltem Bioethanol notwendig sein. Das ethanolhaltige Reinigungsmittel wird nach seiner Verwendung zur Reinigung des Molekularsiebs vorzugsweise in Schritt (c) des ersten Verfahrens verwendet. Das ethanolhaltige Reinigungsmittel kann der fermentierten Brühe zugegeben werden, bevorzugt nach einem zumindest teilweisen Abtrennen von Wasser und/oder einer Schlempe von der fermentierten Brühe, insbesondere nach einem teilweisen Destillieren der fermentierten Brühe. Dann kann das im Reinigungsmittel enthaltene Ethanol in Schritt (c) des ersten Verfahrens gemeinsam mit dem in der fermentierten Brühe enthaltenen Bioethanol von der fermentierten Brühe abgetrennt werden, sodass auch das im Reinigungsmittel enthaltene Ethanol zumindest teilweise wiedergewonnen werden kann. Das ethanolhaltige (insbesondere bioethanolhaltige) Reinigungsmittel kann aber auch als zweites Extraktionsmittel eingesetzt werden, vor allem, wenn das Reinigungsmittel Ethanol und Wasser enthält, insbesondere in einem geeigneten Mengenverhältnis. Dies ermöglicht eine unmittelbare Weiterverwendung im kombinierten Verfahren, womit eine Energieersparnis einhergeht, da eine Entwässerung des Ethanols entfallen kann. Das Reinigungsmittel kann vor seiner Zugabe zur fermentierten Brühe bzw. vor seiner Verwendung als zweites Extraktionsmittel gelagert werden, insbesondere in einem Lagerbehälter. Dies ermöglicht es, je nach Bedarf, Verfügbarkeit und/oder Kapazität zumindest einen Teil des gelagerten Reinigungsmittels zu entnehmen. Alternativ oder zusätzlich zur Reinigung des Molekularsiebs mit einem ethanolhaltigen Reinigungsmittel wird das Molekularsieb vorzugsweise mit Kohlenstoffdioxid gereinigt, insbesondere gespült. Hierfür kann zumindest ein Teil des beim Fermentieren in Schritt (b) des ersten Verfahrens entstandenen Kohlenstoffdioxids verwendet werden. Das beim Fermentieren als Nebenprodukt anfallende Kohlenstoffdioxid kann daher im erfindungsgemäß kombinierten Verfahren weiterverwendet werden, was hinsichtlich der Verfahrenseffizienz vorteilhaft ist. Insbesondere ist dabei die durch die erfindungsgemäße Verfahrenskombination ermöglichte CO₂-Verwertung für die Ethanolproduktion in Bezug auf die Nachhaltigkeit der Bioethanolproduktion im Rahmen des kombinierten Gesamtverfahrens sehr wichtig.

Bevorzugt ist es, wenn das Abtrennen zumindest eines Teils des Bioethanols von der fermentierten Brühe in Schritt (c), insbesondere das Trennen der Bioethanol-Wasser-Mischung in Bioethanol und Wasser, ein Kondensieren von gasförmigem Bioethanol und/oder gasförmigem Wasser umfasst. Dabei kann thermische Energie gewonnen werden. Diese thermische Energie kann im kombinierten Verfahren genutzt werden, insbesondere für das Zerkleinern der Ölfrüchte, um das in Schritt (i) bereitgestellte Ölfruchtausgangsprodukt zu erhalten, für das Extrahieren des Ölfruchtausgangsprodukts mit dem ersten Extraktionsmittel in Schritt (ii) und/oder für das Trennen des ersten Extraktionsprodukts in die erste und zweite Mischung in Schritt (iii). Die erfindungsgemäße Kombination der beiden Verfahren kann somit einen wertvollen Beitrag zur Nachhaltigkeit leisten und die Energiebilanz erheblich verbessern. Beispielsweise können 0,4 bis 0,6 t Wasserdampf (100 °C, 1 bar) als thermische Energie gewonnen werden - dies entspricht ca. ein Drittel des Dampfbedarfs für die Verarbeitung einer Tonne eingesetzter Ölfrüchte, insbesondere Sojabohnen.

Das von der fermentierten Brühe abgetrennte Bioethanol kann gesammelt werden, insbesondere in einem ersten Sammelbehälter. Vorzugsweise wird im ersten Verfahren hergestelltes Bioethanol als erstes Extraktionsmittel und/oder als zweites Extraktionsmittel im zweiten Verfahren verwendet, und/oder weitere Anwendungen wie z.B. als Beimischung für Kraftstoffe, in der Kosmetik, als Ausgangsprodukt für Biobased Chemicals usw., verwendet werden.

Die in Schritt (c) des ersten Verfahrens von der fermentierten Brühe abgetrennte Schlempe wird vorzugsweise nachfolgend getrocknet. Das Trocknen erfolgt vorzugsweise bei einer Temperatur von 60 °C oder darüber, bevorzugter von 100 °C oder darüber, besonders bevorzugt bei 100 bis 140 °C. Hierdurch können Flüssigkeiten, insbesondere Wasser, sowie gegebenenfalls flüchtige Substanzen von der Schlempe abgetrennt werden. Bevorzugt wird die Schlempe für 0,1 h oder mehr getrocknet, bevorzugter für 0,15 h oder mehr, besonders bevorzugt für 0,15 bis 0,3 h. Die getrocknete Schlempe kann als hochwertiges Futtermittel, z.B. als DDGS, verwendet werden. Hierfür wird die getrocknete Schlempe vorzugsweise pelletiert. Danach wird sie vorzugsweise abgepackt, etwa in verkaufsfertige Gebinde. Alternativ oder zusätzlich dazu kann die Schlempe, bzw. ein Teil davon, energetisch verwertet werden, etwa durch Umsetzung in Biogas oder Biomethan. Beim Trocknen der Schlempe abgetrenntes, gasförmiges Wasser wird vorzugsweise kondensiert, wobei thermische Energie gewonnen wird. Die gewonnene thermische Energie kann im kombinierten Verfahren genutzt werden, insbesondere für das Konditionieren beim Zerkleinern der Ölfrüchte, um das in Schritt (i) bereitgestellte Ölfruchtausgangsprodukt zu erhalten, für das Extrahieren des Ölfruchtausgangsprodukts mit dem ersten Extraktionsmittel in Schritt (ii) und/oder für das Trennen des ersten Extraktionsprodukts in die erste und zweite Mischung in Schritt (iii). Die erfindungsgemäße Kombination der beiden Verfahren kann somit einen wertvollen Beitrag zur Nachhaltigkeit leisten und die Energiebilanz erheblich verbessern. Das abgetrennte Wasser wird vorzugsweise aufbereitet, sodass es wiederverwendet werden kann.

Die Schlempe wird vorzugsweise gemeinsam mit Schalen von Ölfrüchten getrocknet. Die Schalen sind vorzugsweise ein Teil von in Schritt (i) des zweiten Verfahrens von Ölfrüchten, insbesondere Sojabohnen, abgetrennten Schalen. Diese Schalen können der in Schritt (c) des ersten Verfahrens von der fermentierten Brühe abgetrennten Schlempe zugegeben werden. Alternativ oder zusätzlich kann die Schlempe bereits zerkleinerte Schalen enthalten, wenn das in der Maische enthaltene kohlenhydrathaltiges Ausgangsmaterial gemeinsam mit pflanzlichen Materialien, insbesondere mit Schalen von Ölfrüchten, Getreide, oder Rübenschnitzel, insbesondere Sojabohnen, zerkleinert wurde. Die Anwesenheit (zerkleinerter) Schalen von Ölfrüchten beim Trocknen der Schlempe bietet prozesstechnische Vorteile: das Trocknen der Schlempe kann dann effizienter erfolgen, da die (zerkleinerten) Schalen Wasser gut absorbieren können, insbesondere dann, wenn sie faserig sind. Auch geht mit dem Zusatz der Schalen zur Schlempe eine Aufwertung der Schalen einher, da die Schalen am Markt ansonsten nur schwer absetzbar sind.

Bevorzugt ist es, wenn der von der fermentierten Brühe abgetrennten Schlempe ein Teil des im zweiten Verfahren erhaltenen Ölfruchtnebenprodukts zugegeben wird, wobei die Schlempe und das zugegebene Ölfruchtnebenprodukt vorzugsweise gemeinsam getrocknet werden. Dies ist vor allem dann vorteilhaft, wenn die im zweiten Verfahren anfallende Menge an Ölfruchtnebenprodukt zu groß ist, um sie gänzlich bereits in Schritt (a) des ersten Verfahrens zu verwenden und dann zusammen mit dem kohlenhydrathaltigen Ausgangsmaterial zu fermentieren; wenn das Verhältnis von Ölfruchtnebenprodukt zu kohlenhydrathaltigem Ausgangsmaterial in der Maische zu groß wäre, könnte dies die Fermentationseigenschaften der Maische verschlechtern. Der Zusatz eines Teils des Ölfruchtnebenprodukts zur Schlempe bietet den Vorteil, dass auch zumindest ein Teil des Ölfruchtnebenprodukts, der nicht fermentiert werden kann (insbesondere aufgrund einer zu großen Menge an anfallendem Ölfruchtnebenprodukt), dennoch im kombinierten Verfahren weiterverwendet werden kann und zu einem wertvollen Futtermittel weiter verarbeitet werden kann.

### Zweites Verfahren - Herstellung des Ölfruchtproteinprodukts

Das Ölfruchtproteinprodukt ist vorzugsweise ein Ölfruchtproteinisolat und/oder ein Ölfruchtproteinkonzentrat, besonders bevorzugt ein Sojaproteinisolat und/oder ein Sojaproteinkonzentrat. Ölfruchtproteinisolat ist ein hochreines Protein, das aus Ölfrüchten gewonnen wird. Normalerweise enthält Ölfruchtproteinisolat mindestens 90 Gew% Protein(e) und einen vergleichsweise geringen Anteil eines Kohlenhydrates und/oder Fettes. Ölfruchtproteinkonzentrat hat eine ähnliche Zusammensetzung wie Ölfruchtproteinisolat, kann allerdings auch einen geringeren Proteingehalt aufweisen (ca. 40 bis 90 Gew%, vorzugsweise 50 bis 90 Gew%, insbesondere 60 bis 80 Gew%), und einen höheren Anteil an Fasern, eines Kohlenhydrates und/oder Fettes verglichen mit Ölfruchtproteinisolat.

Das im zweiten Verfahren anfallende Ölfruchtnebenprodukt enthält vorzugsweise ein Kohlenhydrat, bzw. eine Mischung von zwei oder mehr Kohlenhydraten, wobei vorzugsweise zumindest ein Kohlenhydrat, insbesondere zumindest eine Stärke, verzuckerbar ist. Vorzugsweise enthält das Ölfruchtnebenprodukt zumindest 15 Gew% eines derartigen, verzuckerbaren Kohlenhydrats, bevorzugter zumindest 20 Gew%, noch bevorzugter zumindest 30 Gew%, besonders bevorzugt zumindest 40 Gew%, bezogen auf den Anteil an Kohlenhydraten im Ölfruchtnebenprodukt, und bezogen auf das Trockensubstanzgewicht des Ölfruchtnebenprodukts. Abhängig vom Ausgangsmaterial sind auch höhere Werte möglich, insbesondere wenn kurzkettige Kohlehydrate, z.B. Stachyose und Raffinose, enthalten sind. Unter einem verzuckerbaren Kohlenhydrat wird dabei ein Kohlenhydrat (insbesondere eine Stärke) verstanden, das durch Maischen aufgeschlossen (d.h. verzuckert) und dadurch fermentierbar gemacht werden kann. Dann kann in Schritt (b) des ersten Verfahrens eine Fermentation des durch Maischen entstandenen Zuckers (d.h. eine Umwandlung des Zuckers in Ethanol) erfolgen. Wenn beispielsweise das Ölfruchtnebenprodukt ca. 30 Gew% verzuckerbare Kohlenhydrate enthält (bezogen auf den Anteil an Kohlenhydraten im Ölfruchtnebenprodukt und auf das Trockensubstanzgewicht des Ölfruchtnebenprodukts), kann durch das kombinierte Verfahren die Menge an mittels des ersten Verfahrens hergestelltem Bioethanol um rund 2500 m³ pro Jahr gesteigert werden, wenn das Verhältnis von Ölfruchtnebenprodukt zu kohlenhydrathaltigem Ausgangsmaterial in der Maische bei 1:10 liegt (Gew%/Gew%). Bevorzugt ist es, wenn das im zweiten Verfahren anfallende Ölfruchtnebenprodukt auch ein Protein (bzw. eine Mischung von zwei oder mehr Proteinen) enthält. Da Proteine nicht fermentierbar sind, sind sie in der von der fermentierten Brühe abgetrennten Schlempe enthalten. Die Schlempe ist somit besonders proteinreich, sodass sie als hochwertiges Futtermittel verwendet werden kann.

In Schritt (i) des zweiten Verfahrens wird ein Ölfruchtausgangsprodukt bereitgestellt. Das Ölfruchtausgangsprodukt wird vorzugsweise aus Ölfrüchten erhalten. Vorzugsweise umfasst das Bereitstellen des Ölfruchtausgangsprodukts ein Aufbereiten der Ölfrüchte, insbesondere ein Abtrennen von Schalen der Ölfrüchte und/oder ein Zerkleinern der Ölfrüchte. Beispiele für Ölfrüchte sind Rapskörner, Sonnenblumenkörner, Leinsamen, Senfkörner, Erdnusssamen, Weintraubenkerne, Kürbiskerne, Sojabohnen, Kokosnüsse, Avocados, Kaffeebohnen, Ölpalmensamen, Ölpalmenfrüchte, Olivenkerne und Baumwollsamen. Die Verarbeitbarkeit von Ölfrüchten mittels Extraktion mit alkoholbasierten Lösungsmitteln ist aus der WO 2022/006165 A1 und der WO 2022/183031 A1 bekannt. Bevorzugt sind die Ölfrüchte Sojabohnen, Sonnenblumenkörner und/oder Rapskörner, besonders bevorzugt Sojabohnen. Hiermit lässt sich im zweiten Verfahren eine besonders hohe Ausbeute an Ölfruchtproteinprodukt erreichen. Das Ölfruchtausgangsprodukt enthält dementsprechend vorzugsweise zerkleinerte und/oder geschälte Ölfrüchte; bevorzugt zerkleinerte und/oder geschälte Sojabohnen, zerkleinerte und/oder geschälte Sonnenblumenkörner, und/oder zerkleinerte und/oder geschälte Rapskörner; besonders bevorzugt zerkleinerte und/oder geschälte Sojabohnen. Werden Sojabohnen, insbesondere zerkleinerte und/oder geschälte Sojabohnen, als Ölfruchtausgangsprodukt verwendet, kann im zweiten Verfahren ein Sojaproteinkonzentrat (SPC) und/oder ein Sojaproteinisolat (SPI) als Ölfruchtproteinprodukt erhalten werden, wobei eine Sojamelasse und/oder ein Sojaproteinliquor (SPL) als Ölfruchtnebenprodukt anfallen können.

Zumindest ein Teil der von den Ölfrüchten abgetrennten Schalen wird vorzugsweise bei der Herstellung der in Schritt (a) des ersten Verfahrens bereitgestellten Maische verwendet und/oder gemeinsam mit der Schlempe getrocknet. Die Weiterverwendung abgetrennter Schalen im ersten Verfahren ermöglicht eine höherwertige Verwertung dieser Schalen. Das Zerkleinern, insbesondere Mahlen und/oder Pressen, der Ölfrüchte erfolgt vorzugsweise bei erhöhter Temperatur. Vorzugsweise erfolgt das Zerkleinern bei einer Temperatur von 50 °C oder darüber, bevorzugter von 70 °C oder darüber, noch bevorzugter von 90 °C oder darüber, besonders bevorzugt von 110 °C oder darüber. Hierdurch können die Ölfrüchte zugleich auch (nach-)getrocknet werden, insbesondere kann gegebenenfalls in den Ölfrüchten noch enthaltenes Wasser abgetrennt werden. Dies kann die Sprödigkeit der Ölfrüchte erhöhen und folglich die Effizienz des Zerkleinerns verbessern. Die für das Zerkleinern und/oder Pressen der Ölfrüchte benötigte thermische Energie ist vorzugsweise zumindest zum Teil im kombinierten Verfahren gewonnene thermische Energie, insbesondere beim Abtrennen zumindest eines Teils des Bioethanols von der fermentierten Brühe in Schritt (c) gewonnene thermische Energie (insbesondere beim Kondensieren des Bioethanols nach dem Trennen der Bioethanol-Wasser-Mischung in Bioethanol und Wasser gewonnene thermische Energie), und/oder beim Kondensieren des beim Trocknen der Schlempe abgetrennten Wassers gewonnene thermische Energie. Durch die Nutzung von im kombinierten Verfahren gewonnener thermischer Energie lässt sich die Energiebilanz des kombinierten Verfahrens erheblich verbessern.

In Schritt (ii) des zweiten Verfahrens wird das Ölfruchtausgangsprodukt mit einem ersten Extraktionsmittel extrahiert, um eine erste Miscella und ein erstes Extraktionsprodukt zu erhalten. Unter"Miscella" (lat. Gemischtes) wird erfindungsgemäß-wie auch in der Fachwelt - ein Stoffgemisch aus organischem Extraktionsmittel (Lösungsmittel) und Öl verstanden bezeichnet, das während der Gewinnung von Pflanzenölen als Zwischenprodukt anfällt. Diese Extraktion dient vorzugsweise der Entfettung und/oder Entölung des Ölfruchtausgangsprodukts. Das erste Extraktionsprodukt enthält daher vorzugsweise einen zumindest teilweise entfetteten und/oder entölten Feststoff. Das im Ölfruchtausgangsprodukt enthaltene Öl und/oder Fett ist dementsprechend vorzugsweise zumindest teilweise, bevorzugter zum Großteil (d.h. zu 51 Gew% oder darüber, vorzugsweise zu 80 Gew% oder darüber, bevorzugter zu 90 Gew% oder darüber, noch bevorzugter zu 95 Gew% oder darüber, bezogen auf die im Ölfruchtausgangsprodukt gesamt vorhandene Menge des Öls und/oder Fetts), besonders bevorzugt zur Gänze, in der ersten Miscella enthalten.

Das Extrahieren in Schritt (ii) des zweiten Verfahrens wird vorzugsweise bei erhöhter Temperatur durchgeführt, bevorzugt bei 50°C oder darüber, bevorzugter bei 60°C oder darüber, noch bevorzugter bei 65°C bis 100°C, insbesondere bei 70 bis 90°C. Dadurch kann das Entölen und/oder Entfetten schneller und vollständiger erfolgen. Die für das Extrahieren in Schritt (ii) benötigte thermische Energie ist vorzugsweise zumindest zum Teil im kombinierten Verfahren gewonnene thermische Energie, bevorzugt beim Abtrennen zumindest eines Teils des Bioethanols von der fermentierten Brühe in Schritt (c) gewonnene thermische Energie (insbesondere beim Kondensieren des Bioethanols nach dem Trennen der Bioethanol-Wasser-Mischung in Bioethanol und Wasser gewonnene thermische Energie), und/oder beim Kondensieren des beim Trocknen der Schlempe abgetrennten Wassers gewonnene thermische Energie. Durch die Nutzung von im kombinierten Verfahren anfallender thermischer Energie lässt sich die Energiebilanz des kombinierten Verfahrens erheblich verbessern.

Das erste Extraktionsmittel ist vorzugsweise Ethanol wobei das Ethanol vorzugsweise eine Reinheit von 99,0 Gew% oder darüber aufweist, bevorzugter von 99,5 Gew% oder darüber, besonders bevorzugt von 99,9 Gew% oder darüber (bezogen auf das Gewicht des ersten Extraktionsmittels). Durch den Einsatz von Ethanol, insbesondere mit der genannten Reinheit, ist eine effiziente Entfettung bzw. Entölung des Ölfruchtausgangsprodukts möglich; wird Bioethanol verwendet, kann dies außerdem die Nachhaltigkeit verbessern. Vorzugsweise enthält das erste Extraktionsmittel im ersten Verfahren hergestelltes Bioethanol, insbesondere in Schritt (c) des ersten Verfahrens abgetrenntes Bioethanol; besonders bevorzugt ist das erste Extraktionsmittel im ersten Verfahren hergestelltes Bioethanol. Somit kann an Ort und Stelle hergestelltes Bioethanol verwendet werden, ohne dass Transportkosten anfallen. Diese zusätzliche Verlinkung der beiden kombinierten Verfahren ermöglicht eine weitere Verbesserung der Verfahrenseffizienz und Nachhaltigkeit.

Bevorzugt wird zumindest ein Teil des in der ersten Miscella enthaltenen Öls und/oder Fetts von der ersten Miscella abgetrennt. Dieses Abtrennen umfasst vorzugsweise ein Destillieren und/oder Strippen. Dadurch kann abgetrenntes Öl und/oder Fett rückgewonnen werden und für andere Anwendungsgebiete zum Einsatz kommen. Das Destillieren ist vorzugsweise ein fraktioniertes Destillieren. Dadurch kann eine Trennung einzelner Kohlenstofffraktionen voneinander erfolgen, was deren unmittelbare Weiterverwendung, beispielsweise als Kraftstoff, ermöglicht.

Es ist bevorzugt, wenn zumindest ein Teil des in der ersten Miscella enthaltenen, ersten Extraktionsmittels von der ersten Miscella abgetrennt wird, vorzugsweise durch Destillieren und/oder Strippen. Das zumindest teilweise Abtrennen des ersten Extraktionsmittels von der ersten Miscella ermöglicht dessen Wiederverwendung im kombinierten Verfahren. Das Abtrennen zumindest eines Teils des Öls und/oder Fetts (einschließlich der Trennung in einzelne Kohlenstofffraktionen) sowie das Abtrennen zumindest eines Teils des ersten Extraktionsmittels von der ersten Miscella können in derselben Destillationsapparatur bzw. im selben Stripper erfolgen. Das Abtrennen zumindest eines Teils des Öls und/oder Fetts sowie zumindest eines Teils des ersten Extraktionsmittels von der ersten Miscella sind unabhängig von den Schritten (iii) und (iv) des zweiten Verfahrens. Daher kann dieses Abtrennen parallel zum Schritt (iii) und/oder (iv) des zweiten Verfahrens durchgeführt werden, oder auch im Anschluss an den Schritt (iii) oder (iv).

Das Abtrennen zumindest eines Teils des ersten Extraktionsmittels und gegebenenfalls auch zumindest eines Teils des Öls und/oder Fetts von der ersten Miscella erfolgt vorzugsweise bei einer erhöhten Temperatur, bei 50°C oder darüber, bevorzugter bei 60°C oder darüber, noch bevorzugter bei 65°C bis 100°C, insbesondere bei 70 bis 90°C Die für das Abtrennen benötigte thermische Energie ist vorzugsweise zumindest zum Teil im kombinierten Verfahren gewonnene thermische Energie, bevorzugt beim Abtrennen zumindest eines Teils des Bioethanols von der fermentierten Brühe in Schritt (c) gewonnene thermische Energie (insbesondere beim Kondensieren des Bioethanols nach dem Trennen der Bioethanol-Wasser-Mischung in Bioethanol und Wasser gewonnene thermische Energie), und/oder beim Kondensieren des beim Trocknen der Schlempe abgetrennten Wassers gewonnene thermische Energie.

Wenn das erste Extraktionsmittel Ethanol ist, weist das abgetrennte, erste Extraktionsmittel vorzugsweise einen Ethanolgehalt von 85 Gew% oder darüber auf, bevorzugter von 90 Gew% oder darüber, noch bevorzugter von 95 Gew% oder darüber, bezogen auf das Gewicht des abgetrennten, ersten Extraktionsmittels. Der Ethanolgehalt im abgetrennten, ersten Extraktionsmittel kann etwas niedriger sein als der Ethanolgehalt des ersten Extraktionsmittels (dieses weist vorzugsweise eine Reinheit von 99,0 Gew% oder darüber auf, wie oben ausgeführt); insbesondere kann Wasser gemeinsam mit dem ersten Extraktionsmittel abgetrennt werden.

Vorzugsweise wird das von der ersten Miscella abgetrennte, erste Extraktionsmittel (enthaltend Ethanol) in Schritt (c) des ersten Verfahrens verwendet, bevorzugt wird es der in Schritt (b) des ersten Verfahrens erhaltenen, fermentierten Brühe zugegeben. Vorzugsweise wird das abgetrennte, erste Extraktionsmittel der fermentierten Brühe zugegeben, bevorzugt nach einem zumindest teilweisen Abtrennen von Wasser und/oder einer Schlempe von der fermentierten Brühe, insbesondere nach einem teilweisen Destillieren der fermentierten Brühe. Bei der Zugabe des abgetrennten, ersten Extraktionsmittels zur fermentierten Brühe (vorzugsweise nach deren teilweisem Destillieren) kann der Ethanolgehalt, der fermentierten Brühe vorzugsweise um 1 Gew% oder darüber, bevorzugter um 1,5 Gew% oder darüber, noch bevorzugter um 2 Gew% oder darüber, erhöht werden. Dadurch kann das Abtrennen, insbesondere Destillieren, in Schritt (c) möglichst effizient erfolgen, zumal der Ethanolgehalt des abgetrennten, ersten Extraktionsmittels höher sein kann als der Ethanolgehalt der fermentierten Brühe unmittelbar nach deren Fermentation. Die Verwendung des abgetrennten, ersten Extraktionsmittels in Schritt (c) des ersten Verfahrens ermöglicht es, zumindest ein Teil des im ersten Extraktionsmittel enthaltenen Ethanols, gemeinsam mit zumindest einem Teil des in Schritt (b) durch Fermentieren erhaltenen Bioethanols von der fermentierten Brühe abzutrennen. Ein separater Entwässerungsschritt des ersten Extraktionsmittels kann somit entfallen, da dies im Zuge Schritt (c) durchgeführt wird. Dies reduziert die Anschaffungskosten für den zweiten Prozess. Vorzugsweise wird im ersten Verfahren hergestelltes Bioethanol als erstes Extraktionsmittel verwendet, und zumindest ein Teil des ersten Extraktionsmittels wird in Schritt (c) des ersten Verfahrens verwendet. Somit kann (Bio-)Ethanol im kombinierten Verfahren zirkulieren. Alternativ oder zusätzlich dazu ist es bevorzugt, wenn das abgetrennte, erste Extraktionsmittel (enthaltend Bioethanol) zum Trennen des ersten Extraktionsprodukts in die erste Mischung und in die zweite Mischung verwendet wird, insbesondere als zweites Extraktionsmittel. Wenn notwendig, kann Wasser zugegeben werden, um den Ethanolgehalt entsprechend dem für das zweite Extraktionsmittel geeigneten Ethanolgehalt anzupassen. Auch dadurch wird eine Zirkulation von (Bio-)Ethanol innerhalb des erfindungsgemäßen, kombinierten Verfahrens ermöglicht. Diese Zirkulation trägt maßgeblich zur Verbesserung der Verfahrenseffizienz und Nachhaltigkeit des kombinierten Verfahrens bei.

Das von der ersten Miscella abgetrennte, erste Extraktionsmittel (enthaltend Ethanol, vorzugsweise Bioethanol) kann vor seiner Verwendung im ersten Verfahren gelagert werden, insbesondere im Lagerbehälter, gegebenenfalls zusammen mit dem zur Reinigung des Molekularsiebs (in Schritt (c) des ersten Verfahrens) verwendeten Reinigungsmittel (ebenfalls enthaltend Ethanol, vorzugsweise Bioethanol). Dies ermöglicht es, je nach Bedarf, Verfügbarkeit und/oder Kapazität zumindest einen Teil des gelagerten ersten Extraktionsmittels, bzw. einer Mischung des gelagerten ersten Extraktionsmittels und des gelagerten Reinigungsmittels, zur Weiterverwendung im kombinierten Verfahren zu entnehmen.

In Schritt (iii) des zweiten Verfahrens wird das erste Extraktionsprodukt in die erste Mischung enthaltend das Ölfruchtproteinprodukt und in die zweite Mischung enthaltend das Ölfruchtnebenprodukt getrennt. Die Ausgestaltung des zweiten Verfahrens kann nach Schritt (ii) in Abhängigkeit des herzustellenden Ölfruchtproteinprodukts variieren, insbesondere kann für die Herstellung des Ölfruchtproteinisolats sowie des Ölfruchtproteinkonzentrats eine jeweils unterschiedliche Verfahrensführung erforderlich sein.

Im zweiten Verfahren kann auch eine gleichzeitige Herstellung des Ölfruchtproteinisolats und des Ölfruchtproteinkonzentrats erfolgen. Dies kann dadurch realisiert werden, indem ein erster Teil des ersten Extraktionsprodukts zur Herstellung des Ölfruchtproteinkonzentrats und ein zweiter Teil des ersten Extraktionsprodukts zur Herstellung des Ölfruchtproteinisolats verwendet werden. Die Möglichkeit der Herstellung beider dieser Ölfruchtproteinprodukte erhöht die Flexibilität des kombinierten Verfahrens, zumal je nach Bedarf bzw. Auftragslage entweder beide Ölfruchtproteinprodukte gleichzeitig hergestellt werden können, oder nur eines davon.

Wenn das herzustellende Ölfruchtproteinprodukt ein Ölfruchtproteinkonzentrat, insbesondere ein Sojaproteinkonzentrat, ist, wird das erste Extraktionsprodukt in Schritt (iii) vorzugsweise mit einem zweiten Extraktionsmittel extrahiert, um es in die erste Mischung und die zweite Mischung zu trennen. Die zweite Mischung wird auch als zweite Miscella bezeichnet. Die erste Mischung enthält das Ölfruchtproteinkonzentrat sowie gegebenenfalls eine geringe Menge an verbliebenem zweiten Extraktionsmittel. Die zweite Mischung enthält ein Ölfruchtnebenprodukt, das die Ölfruchtmelasse enthält. Die Ölfruchtmelasse enthält typischerweise Kohlenhydrate, einen geringen Anteil an Proteinen, Fette und Fasern. Weiters enthält die zweite Mischung den Großteil des zweiten Extraktionsmittels.

Das Extrahieren in Schritt (iii) des zweiten Verfahrens wird vorzugsweise bei erhöhter Temperatur durchgeführt, bevorzugt bei 25°C oder darüber, bevorzugter bei 30°C oder darüber, noch bevorzugter bei 35 bis 50°C. Dadurch kann das Trennen des ersten Extraktionsprodukts in die erste Mischung und die zweite Mischung schneller und besser erfolgen. Die für das Extrahieren in Schritt (iii) benötigte thermische Energie ist vorzugsweise zumindest zum Teil im kombinierten Verfahren gewonnene thermische Energie, bevorzugt beim Abtrennen zumindest eines Teils des Bioethanols von der fermentierten Brühe in Schritt (c) gewonnene thermische Energie (insbesondere beim Kondensieren des Bioethanols nach dem Trennen der Bioethanol-Wasser-Mischung in Bioethanol und Wasser gewonnene thermische Energie), und/oder beim Kondensieren des beim Trocknen der Schlempe abgetrennten Wassers gewonnene thermische Energie. Durch die Nutzung von im kombinierten Verfahren gewonnener thermischer Energie lässt sich die Energiebilanz des kombinierten Verfahrens erheblich verbessern.

Das zweite Extraktionsmittel enthält vorzugsweise Ethanol (insbesondere Bioethanol) und Wasser (z.B. 20 bis 90 Gew% oder 40 bis 90 Gew%; Peng et al., Foods 10 (2021), 2222), wobei der Ethanolgehalt vorzugsweise im Bereich von 55 bis 90 Gew% liegt, bevorzugter von 60 bis 80 Gew%, bezogen auf das Gewicht des zweiten Extraktionsmittels. Bevorzugt enthält das im zweiten Extraktionsmittel enthaltene Ethanol im ersten Verfahren hergestelltes Bioethanol, insbesondere in Schritt (c) des ersten Verfahrens abgetrenntes Bioethanol; besonders bevorzugt ist das im zweiten Extraktionsmittel enthaltene Ethanol im ersten Verfahren hergestelltes Bioethanol. Diese zusätzliche Verlinkung der beiden kombinierten Verfahren ermöglicht eine weitere Verbesserung der Verfahrenseffizienz. Durch die Extraktion mit dem zweiten Extraktionsmittel können Kohlenhydrate aus dem ersten Extraktionsprodukt extrahiert werden. Diese Kohlenhydrate sind dann vorzugsweise zumindest teilweise, bevorzugter zum Großteil, in der zweiten Miscella enthalten.

Es ist bevorzugt, wenn zumindest ein Teil des zweiten Extraktionsmittels von der zweiten Mischung abgetrennt wird, vorzugsweise durch Destillieren und/oder Strippen. Das zumindest teilweise Abtrennen des zweiten Extraktionsmittels ermöglicht dessen Wiederverwendung. Einerseits kann das abgetrennte, zweite Extraktionsmittel nachfolgend wieder als zweites Extraktionsmittel verwendet werden. Das Abtrennen zumindest eines Teils des zweiten Extraktionsmittels von der zweiten Mischung erfolgt vorzugsweise bei einer erhöhten Temperatur, bevorzugt bei 25°C oder darüber, bevorzugter bei 30°C oder darüber, noch bevorzugter bei 35 bis 50°C. Die für das Abtrennen benötigte thermische Energie ist vorzugsweise zumindest zum Teil im kombinierten Verfahren gewonnene thermische Energie, bevorzugt beim Abtrennen zumindest eines Teils des Bioethanols von der fermentierten Brühe in Schritt (c) gewonnene thermische Energie (insbesondere beim Kondensieren des Bioethanols nach dem Trennen der Bioethanol-Wasser-Mischung in Bioethanol und Wasser gewonnene thermische Energie), und/oder beim Kondensieren des beim Trocknen der Schlempe abgetrennten Wassers gewonnene thermische Energie.

Wenn das zweite Extraktionsmittel Ethanol (insbesondere Bioethanol) enthält, weist das von der zweiten Mischung abgetrennte, zweite Extraktionsmittel vorzugsweise einen Ethanolgehalt von 55 Gew% oder darüber auf, bevorzugter von 60 Gew% oder darüber, noch bevorzugter von 65 Gew% oder darüber, besonders bevorzugt von 70 Gew% oder darüber, bezogen auf das Gewicht des abgetrennten, zweiten Extraktionsmittels; der restliche Anteil (um 100 Gew% zu erhalten) ist vorzugsweise Wasser. Hierdurch kann ein geeignetes Mengenverhältnis von Ethanol zu Wasser erhalten werden, um die zweite Extraktion effizient durchzuführen. Wird das abgetrennte, zweite Extraktionsmittel, das Ethanol und Wasser enthält, als zweites Extraktionsmittel wiederverwendet, kann eine Entwässerung des Ethanols entfallen. Zumindest ein Teil des zweiten Extraktionsmittels wird vorzugsweise als zweites Extraktionsmittel wiederverwendet. Gegebenenfalls kann das abgetrennte, zweite Extraktionsmittel vor der Wiederverwendung als zweites Extraktionsmittel gereinigt werden, wobei das Reinigen vorzugsweise ein Filtrieren umfasst. Andererseits kann das abgetrennte, zweite Extraktionsmittel, wenn es Ethanol (insbesondere Bioethanol) und Wasser enthält, in Schritt (c) des ersten Verfahrens verwendet werden. Vorzugsweise wird das abgetrennte, zweite Extraktionsmittel der in Schritt (b) des ersten Verfahrens erhaltenen, fermentierten Brühe zugegeben, bevorzugt nach einem zumindest teilweisen Abtrennen von Wasser und/oder einer Schlempe von der fermentierten Brühe, insbesondere nach einem teilweisen Destillieren der fermentierten Brühe. Dann kann in Schritt (c) des ersten Verfahrens zumindest ein Teil des als zweites Extraktionsmittel verwendeten Ethanols zusammen mit zumindest einem Teil des in Schritt (b) durch Fermentieren erhaltenen Bioethanols von der fermentierten Brühe abgetrennt werden. Dementsprechend kann eine separate Entwässerung des abgetrennten zweiten Extraktionsmittels entfallen, was verfahrenstechnisch vorteilhaft ist. Besonders bevorzugt wird im ersten Verfahren hergestelltes Bioethanol als zweites Extraktionsmittel verwendet, und zumindest ein Teil des zweiten Extraktionsmittels wird anschließend in Schritt (c) des ersten Verfahrens verwendet. Dadurch kann eine Zirkulation von Bioethanol im kombinierten Verfahren realisiert werden, was die Nachhaltigkeit und Verfahrenseffizienz weiter verbessert.

Das abgetrennte, zweite Extraktionsmittel (enthaltend Ethanol und Wasser) kann vor seiner Wiederverwendung als zweites Extraktionsmittel und/oder vor seiner Verwendung in Schritt (c) des ersten Verfahrens gelagert werden, insbesondere im Lagerbehälter. Dies ermöglicht es, je nach Bedarf, Verfügbarkeit und/oder Kapazität zumindest einen Teil des gelagerten zweiten Extraktionsmittels zu entnehmen. Wird auch das abgetrennte, erste Extraktionsmittel (enthaltend Ethanol) gelagert, können das abgetrennte, erste Extraktionsmittel, das abgetrennte, zweite Extraktionsmittel, und gegebenenfalls das zur Reinigung des Molekularsiebs (in Schritt (c) des ersten Verfahrens) verwendete Reinigungsmittel (enthaltend Ethanol), zusammen gelagert werden, insbesondere, wenn sowohl das abgetrennte erste als auch das zweite Extraktionsmittel Ethanol enthalten.

Wenn das herzustellende Ölfruchtproteinprodukt ein Ölfruchtproteinisolat, insbesondere ein Sojaproteinisolat, ist, wird zwischen den Schritten (ii) und (iii) vorzugsweise zumindest ein Teil des im ersten Extraktionsprodukt enthaltenen ersten Extraktionsmittels vom ersten Extraktionsprodukt abgetrennt. Dieses Abtrennen erfolgt vorzugsweise mittels Verdampfen. Durch das zumindest teilweise Abtrennen des ersten Extraktionsmittels, insbesondere Ethanols, vom ersten Extraktionsprodukt kann das erste Extraktionsprodukt im nachfolgenden Schritt (iii) besser in die erste und zweite Mischung getrennt werden. Die für das Verdampfen benötigte thermische Energie ist vorzugsweise zumindest zum Teil im kombinierten Verfahren gewonnene thermische Energie, bevorzugt beim Abtrennen zumindest eines Teils des Bioethanols von der fermentierten Brühe in Schritt (c) gewonnene thermische Energie, und/oder beim Kondensieren des beim Trocknen der Schlempe abgetrennten Wassers gewonnene thermische Energie. Dadurch lässt sich die Energiebilanz des kombinierten Verfahrens erheblich verbessern. Das verdampfte, erste Extraktionsmittel kann nachfolgend kondensiert werden. Die hierbei gewonnene thermische Energie kann ebenfalls im kombinierten Verfahren verwendet werden. Das vom ersten Extraktionsprodukt abgetrennte, erste Extraktionsmittel wird, wenn es Ethanol (insbesondere Bioethanol) enthält, vorzugsweise in Schritt (c) des ersten Verfahrens verwendet. Vorzugsweise wird das abgetrennte, erste Extraktionsmittel der in Schritt (b) des ersten Verfahrens erhaltenen, fermentierten Brühe zugegeben, bevorzugt nach einem zumindest teilweisen Abtrennen von Wasser und/oder einer Schlempe von der fermentierten Brühe, insbesondere nach einem teilweisen Destillieren der fermentierten Brühe. Dann kann in Schritt (c) des ersten Verfahrens zumindest ein Teil des als ersten Extraktionsmittel verwendeten Ethanols, insbesondere Bioethanols, zusammen mit zumindest einem Teil des in Schritt (b) durch Fermentieren erhaltenen Bioethanols von der (vorzugsweise teilweise destillierten) fermentierten Brühe abgetrennt werden. Somit kann (Bio-)Ethanol im kombinierten Verfahren zirkulieren, was zur Verbesserung der Verfahrenseffizienz und Nachhaltigkeit des kombinierten Verfahrens beiträgt.

Wenn das herzustellende Ölfruchtproteinprodukt ein Ölfruchtproteinisolat ist, wird vorzugsweise in Schritt (iii) Wasser zum ersten Extraktionsprodukt zugegeben, der pH-Wert vorzugsweise auf 7-9,5 , bevorzugt auf 9 gestellt, der nicht-gelöste Rückstand abgetrennt und daraufhin wird der pH-Wert des ersten, nun gelösten Extraktionsprodukts, im Wesentlichen auf den isoelektrischen Punkt, vorzugsweise bei pH 3 bis pH 5, des Ölfruchtproteinprodukts eingestellt, um zumindest einen Teil des Ölfruchtproteinisolats auszufällen. Dabei wird somit zunächst neutral-alkalisch extrahiert, der Rückstand abgetrennt, und schließlich isoelektrisch gefällt, gegebenenfalls mit entsprechender pH-Einstellung. Dadurch kann das erste Extraktionsprodukt in die erste (ausgefällte) Mischung und in die zweite Mischung getrennt werden. Die erste Mischung enthält das Ölfruchtproteinisolat sowie gegebenenfalls eine geringe Menge des in Schritt (iii) vorgehend zugegebenen Wassers. Die zweite Mischung enthält das Ölfruchtnebenprodukt, das den Ölfruchtproteinliquor enthält. Der Ölfruchtproteinliquor enthält typischerweise Kohlenhydrate, Proteine und Fette, jedoch (im Gegensatz zur Ölfruchtmelasse) kein Okara (bzw. nur einen vergleichsweise geringen Anteil an Okara) Der Fettanteil dieses Produktes ist daher vorzugsweise verschwindend gering und liegt daher bevorzugt unter 2 Gew%, noch bevorzugter unter 1 Gew%, insbesondere im Bereich von 0,5 bis 0,2 Gew%. Weiters enthält die zweite Mischung vorzugsweise den Großteil des in Schritt (iii) zugegebenen Wassers. Als Okara wird im Allgemeinen ein Nebenprodukt der Sojaproduktherstellung bezeichnet, das aus den Fasern und Proteinen besteht, die aus der Sojabohne bei der Herstellung von Sojaprodukten (wie Sojamilch oder Tofu) übrigbleiben. Es enthält Proteine, Ballaststoffe, Vitamine (Vitamin D), Sojalecithin, Linolsäure, Linolensäure, Phytosterine, Tocopherol sowie Mineralstoffe wie Kalzium, Phosphor und Eisen. Okara besteht zu 76 bis 80 % aus Wasser, 3,5 bis 4,0 % Eiweiß und 20 bis 24 % Feststoffen. Im feuchten Zustand enthält Okara 8 bis 15 % Lipide, 12 bis 14,5 % Rohfasern, 24 % Proteine und 17 % der Proteine der Ausgangssojabohnen.

In Schritt (iv) des zweiten Verfahrens wird zumindest eines Teils des Ölfruchtproteinprodukts, insbesondere Sojaproteinprodukts, von der ersten Mischung abgetrennt, und gegebenenfalls wird zumindest eines Teils des Ölfruchtnebenprodukts, insbesondere Sojanebenprodukts, von der zweiten Mischung abgetrennt.

Wenn das herzustellende Ölfruchtproteinprodukt ein Ölfruchtproteinkonzentrat ist, wird in Schritt (iv) zumindest ein Teil des Ölfruchtproteinkonzentrats von der ersten Mischung, insbesondere vom zweiten Extraktionsmittel, abgetrennt. Das Abtrennen umfasst vorzugsweise ein Verdampfen zumindest eines Teils des zweiten Extraktionsmittels. Die für das Verdampfen benötigte thermische Energie ist vorzugsweise zumindest zum Teil die im kombinierten Verfahren gewonnene thermische Energie, bevorzugt beim Abtrennen zumindest eines Teils des Bioethanols von der fermentierten Brühe in Schritt (c) gewonnene thermische Energie, und/oder beim Kondensieren des beim Trocknen der Schlempe abgetrennten Wassers gewonnene thermische Energie. Dadurch lässt sich die Energiebilanz des kombinierten Verfahrens erheblich verbessern. Das verdampfte, zweite Extraktionsmittel kann kondensiert und nachfolgend wiederverwendet werden. Wenn das zweite Extraktionsmittel Ethanol (insbesondere Bioethanol) enthält, wird abgetrenntes, zweites Extraktionsmittel vorzugsweise der in Schritt (b) des ersten Verfahrens erhaltenen, fermentierten Brühe zugegeben; gegebenenfalls wird es vorzugsweise vorher kondensiert. Dann kann in Schritt (c) des ersten Verfahrens zumindest ein Teil des im zweiten Extraktionsmittel enthaltenen Ethanols, insbesondere Bioethanols, gemeinsam mit zumindest einem Teil des in Schritt (b) des ersten Verfahrens durch Fermentieren erhaltenen Bioethanols von der fermentierten Brühe abgetrennt werden. Das hergestellte Bioethanol kann nachfolgend (wieder) zur Verwendung als erstes und/oder zweites Extraktionsmittel herangezogen werden. Somit kann Bioethanol im kombinierten Verfahren zirkulieren. Vor der Zugabe zur fermentierten Brühe kann das vom Ölfruchtproteinkonzentrat abgetrennte, zweite Extraktionsmittel gelagert werden, insbesondere im Lagerbehälter. Wird das zweite Extraktionsmittel nach dem Abtrennen kondensiert, kann thermische Energie gewonnen werden. Diese kann im kombinierten Verfahren genutzt werden, was die Energiebilanz des kombinierten Verfahrens weiter verbessert.

Das in Schritt (iv) abgetrennte Ölfruchtproteinkonzentrat wird vorzugsweise nachfolgend getrocknet. Dadurch kann etwaiges verbliebenes, zweites Extraktionsmittel entfernt werden. Auch für das Trocknen, das vorzugsweise bei erhöhter Temperatur erfolgt, wird bevorzugt zumindest zum Teil im kombinierten Verfahren gewonnene thermische Energie verwendet. Vorzugsweise enthält das Ölfruchtproteinkonzentrat nach Schritt (iv) des zweiten Verfahrens, insbesondere nach dem Trocknen, eine Menge an zweitem Extraktionsmittel (also insbesondere Ethanol) von 0,1 Gew% oder darunter, bevorzugter von 0,01 Gew% oder darunter, noch bevorzugter von 0,001 Gew% oder darunter, bezogen auf das Gesamtgewicht des Ölfruchtproteinkonzentrats. Das Ölfruchtproteinkonzentrat weist vorzugsweise somit eine hohe Reinheit auf und ist verkaufs- bzw. verzehrfertig. Das Ölfruchtproteinkonzentrat kann nach Schritt (iv) des zweiten Verfahrens, insbesondere nach dem Trocknen, gesammelt werden, insbesondere in einem zweiten Sammelbehälter. Der zweite Sammelbehälter kann eine für den Verkauf geeignete Größe aufweisen, bzw. kann das Ölfruchtproteinkonzentrat nachfolgend in ein geeignetes Gebinde überführt werden. Das hergestellte Ölfruchtproteinkonzentrat wird vorzugsweise als hochwertiges Nahrungsmittel und/oder Futtermittel verwendet.

Wenn das Ölfruchtnebenprodukt eine Ölfruchtmelasse enthält, wird vorzugsweise in Schritt (iv) zumindest ein Teil des Ölfruchtnebenprodukts enthaltend die Ölfruchtmelasse von der zweiten Mischung (insbesondere von der zweiten Miscella) abgetrennt. Das Abtrennen umfasst vorzugsweise ein Strippen und/oder ein Dekantieren. Vorzugsweise wird zumindest ein Teil des abgetrennten Ölfruchtnebenprodukts im ersten Verfahren verwendet und ist in der in Schritt (a) bereitgestellten Maische enthalten, vorzugsweise zumindest teilweise im gemaischten Zustand. Das Ölfruchtnebenprodukt kann vor seiner Verwendung im ersten Verfahren eingedampft werden. Dadurch kann der Wassergehalt des Ölfruchtnebenprodukts verringert werden. Das vom Ölfruchtnebenprodukt abgetrennte Wasser wird vorzugsweise aufbereitet (d.h. gereinigt), sodass es wiederverwendet werden kann.

Das Abtrennen zumindest eines Teils des Ölfruchtnebenprodukts von der zweiten Mischung erfolgt vorzugsweise bei einer erhöhten Temperatur, bevorzugt bei 25°C oder darüber, bevorzugter bei 30°C oder darüber, noch bevorzugter bei 35 bis 50°C. Die für das Abtrennen benötigte thermische Energie ist vorzugsweise zumindest zum Teil im kombinierten Verfahren gewonnene thermische Energie, bevorzugt beim Abtrennen zumindest eines Teils des Bioethanols von der fermentierten Brühe in Schritt (c) gewonnene thermische Energie (insbesondere beim Kondensieren des Bioethanols nach dem Trennen der Bioethanol-Wasser-Mischung in Bioethanol und Wasser gewonnene thermische Energie), und/oder beim Kondensieren des beim Trocknen der Schlempe abgetrennten Wassers gewonnene thermische Energie.

Wenn das Ölfruchtnebenprodukt eine Ölfruchtmelasse enthält, wird vorzugsweise in Schritt (iv) zumindest ein Teil des zweiten Extraktionsmittels von der zweiten Mischung abgetrennt. Das Abtrennen umfasst vorzugsweise ein Destillieren, ein Strippen, ein Zentrifugieren, ein Verdampfen und/oder ein Dekantieren. Das von der zweiten Mischung, insbesondere vom Ölfruchtnebenprodukt enthaltend die Ölfruchtmelasse, abgetrennte zweite Extraktionsmittel kann wiederverwendet werden. Wenn das zweite Extraktionsmittel Ethanol (insbesondere Bioethanol) enthält, wird durch Verdampfen abgetrenntes, zweites Extraktionsmittel vorzugsweise gegebenenfalls kondensiert und in Schritt (c) des ersten Verfahrens verwendet. Vorzugsweise wird das abgetrennte, zweite Extraktionsmittel der in Schritt (b) des ersten Verfahrens erhaltenen, fermentierten Brühe zugegeben, bevorzugt nach einem zumindest teilweisen Abtrennen von Wasser und/oder einer Schlempe von der fermentierten Brühe, insbesondere nach einem teilweisen Destillieren der fermentierten Brühe. Dann kann in Schritt (c) des ersten Verfahrens zumindest ein Teil des im zweiten Extraktionsmittel enthaltenen Ethanols, insbesondere Bioethanols, gemeinsam mit zumindest einem Teil des in Schritt (b) des ersten Verfahrens durch Fermentieren erhaltenen Bioethanols von der (vorzugsweise teilweise destillierten) fermentierten Brühe abgetrennt werden. Das hergestellte Bioethanol kann nachfolgend (wieder) zur Verwendung als erstes und/oder zweites Extraktionsmittel herangezogen werden. Somit kann Bioethanol im kombinierten Verfahren zirkulieren. Vor der Zugabe zur fermentierten Brühe kann das von der zweiten Mischung abgetrennte, zweite Extraktionsmittel gelagert werden, insbesondere im Lagerbehälter.

Zumindest ein Teil der zweiten Mischung kann nach dem Abtrennen zumindest eines Teils des zweiten Extraktionsmittels im ersten Verfahren verwendet werden, und dieser Teil der zweiten Mischung kann in der in Schritt (a) bereitgestellten Maische enthalten sein. Die zweite Mischung kann vor ihrer Verwendung im ersten Verfahren eingedampft werden. Dadurch kann der Wassergehalt der zweiten Mischung verringert werden. Vorzugsweise hat die im ersten Verfahren verwendete zweite Mischung ein Trockensubstanzgewicht von 10 bis 80 Gew%, bevorzugter von 15 bis 50 Gew%, noch bevorzugter von 20 bis 40 Gew%, besonders bevorzugt von 25 bis 35 Gew%, bezogen auf das Gesamtgewicht der zweiten Mischung. Insgesamt kann somit zumindest ein Teil der zweiten Mischung (nachdem zumindest ein Teil des zweiten Extraktionsmittels abgetrennt wurde) und/oder zumindest ein Teil des Ölfruchtnebenprodukts (nach Abtrennen zumindest eines Teils der Ölfruchtnebenprodukts von der zweiten Mischung) im ersten Verfahren verwendet werden und in der in Schritt (a) bereitgestellten Maische enthalten sein. Vorzugsweise wird zumindest ein Teil des Ölfruchtnebenprodukts von der zweiten Mischung abgetrennt und im ersten Verfahren verwendet, sodass dieser Teil des Ölfruchtnebenprodukts in der in Schritt (a) bereitgestellten Maische enthalten ist. Das Abtrennen zumindest eines Teils des Ölfruchtnebenprodukts und zumindest eines Teils des zweiten Extraktionsmittels von der zweiten Mischung, insbesondere das zumindest teilweise Trennen des Ölfruchtnebenprodukts und des zweiten Extraktionsmittels voneinander, erfolgt vorzugsweise in derselben Destillationsapparatur bzw. im selben Stripper. Dies vereinfacht die Verfahrensführung.

Wenn das herzustellende Ölfruchtproteinprodukt ein Ölfruchtproteinisolat, insbesondere ein Sojaproteinisolat, ist, umfasst das Abtrennen zumindest eines Teils des Ölfruchtproteinisolats von der ersten Mischung in Schritt (iv) des zweiten Verfahrens, vorzugsweise ein Entwässern des Ölfruchtproteinisolats, insbesondere ein Verdampfen von Wasser, ein Zentrifugieren, ein Dekantieren und/oder ein Sedimentieren. Vorzugsweise wird das Ölfruchtproteinisolat vor dem Entwässern mit Wasser gewaschen. Hierdurch kann die Reinheit des Ölfruchtproteinisolats verbessert werden. Das abgetrennte Ölfruchtproteinisolat wird vorzugsweise getrocknet, insbesondere sprühgetrocknet. Dadurch kann etwaiges verbliebenes Wasser entfernt werden. In Schritt (iv) von der ersten Mischung, insbesondere vom Ölfruchtproteinisolat, abgetrenntes Wasser wird vorzugsweise aufbereitet, um es wiederverwenden zu können. Vorzugsweise enthält das Ölfruchtproteinisolat nach Schritt (iv) des zweiten Verfahrens, insbesondere nach dem Trocknen, eine Menge an Wasser von 15 Gew% oder darunter, bevorzugter von 10 Gew% oder darunter, noch bevorzugter von 8 Gew% oder darunter, insbesondere von 2 bis 7 Gew%, bezogen auf das Gesamtgewicht des Ölfruchtproteinisolats. Das Ölfruchtproteinisolat weist vorzugsweise somit eine hohe Reinheit auf und ist dementsprechend verkaufs- bzw. verzehrfertig.

Zumindest ein Teil der zweiten Mischung (enthaltend das Ölfruchtnebenprodukt) kann im ersten Verfahren verwendet werden, und dieser Teil der zweiten Mischung kann in der in Schritt (a) bereitgestellten Maische enthalten sein. Vorzugsweise wird aber in Schritt (iv) des zweiten Verfahrens zumindest ein Teil des Ölfruchtnebenprodukts (enthaltend den Ölfruchtproteinliquor) von der zweiten Mischung abgetrennt. Das Abtrennen umfasst vorzugsweise ein Entwässern des Ölfruchtnebenprodukts, insbesondere ein Verdampfen von Wasser, ein Zentrifugieren, ein Dekantieren und/oder ein Sedimentieren. Dadurch kann der Wassergehalt des Ölfruchtnebenprodukts verringert werden. Bevorzugt ist es, wenn das Abtrennen zumindest eines Teils des Ölfruchtnebenprodukts von der zweiten Mischung ein Verdampfen von Wasser umfasst. In Schritt (iv) von der zweiten Mischung, insbesondere vom Ölfruchtnebenprodukt, abgetrenntes Wasser wird vorzugsweise aufbereitet, um es wiederverwenden zu können.

Das im zweiten Verfahren hergestellte Ölfruchtproteinprodukt, insbesondere Sojaproteinprodukt, kann gesammelt werden, insbesondere in einem zweiten Sammelbehälter. Vorzugsweise wird das im zweiten Verfahren hergestellte Ölfruchtproteinprodukt als Futtermittel und/oder als Nahrungsmittel verwendet.

Wenn im zweiten Verfahren das Ölfruchtproteinisolat und das Ölfruchtproteinkonzentrat gleichzeitig und/oder hintereinander (z.B. abwechselnd) hergestellt werden und folglich sowohl ein Ölfruchtnebenprodukt enthaltend die Ölfruchtmelasse als auch ein Ölfruchtnebenprodukt enthaltend den Ölfruchtproteinliquor anfallen, werden vorzugsweise zumindest ein Teil des Ölfruchtnebenprodukts enthaltend die Ölfruchtmelasse und zumindest ein Teil des Ölfruchtnebenprodukts enthaltend den Ölfruchtproteinliquor, bzw. zumindest ein Teil der zweiten Mischung enthaltend das jeweilige Ölfruchtnebenprodukt, gemeinsam im ersten Verfahren verwendet. Die in Schritt (a) des ersten Verfahrens bereitgestellte Maische enthält in diesem Fall den Ölfruchtproteinliquor und die Ölfruchtmelasse. Dadurch können im kombinierten Verfahren anfallende Ölfruchtnebenprodukte bestmöglich verwertet werden.

### Futtermittel

Die Erfindung betrifft weiters ein Futtermittel, erhältlich durch das erfindungsgemäße, kombinierte Verfahren, und enthaltend Schalen von Ölfrüchten, vorzugsweise zerkleinerte Schalen von Ölfrüchten, insbesondere gemahlene Schalen von Ölfrüchten, wobei die Ölfrüchte vorzugsweise Sojabohnen enthalten, wobei die Ölfrüchte insbesondere Sojabohnen, Sonnenblumenkerne und/oder Rapskörner sind. Dadurch geht eine Aufwertung der Schalen der Ölfrüchte einher, da diese am Markt ansonsten nur schwer absetzbar sind und daher oftmals verbrannt werden.

Die Erfindung betrifft weiters ein Futtermittel, erhältlich durch das erfindungsgemäße, kombinierte Verfahren, und enthaltend das Ölfruchtnebenprodukt, insbesondere die Ölfruchtmelasse und/oder den Ölfruchtproteinliquor. Bevorzugt ist die Ölfruchtmelasse eine Sojamelasse, und/oder der Ölfruchtproteinliquor ist ein Sojaproteinliquor.
Zwar wird im kombinierten Verfahren das im zweiten Verfahren entstehende Ölfruchtnebenprodukt im ersten Verfahren verwendet, jedoch kann, wenn die Menge an Ölfruchtnebenprodukt so groß ist, dass aus Kapazitätsgründen nicht die gesamte Menge zusammen mit kohlenhydrathaltigem Ausgangsmaterial gemaischt werden kann, der Rest des Ölfruchtnebenprodukts als Futtermittelzusatz verwendet und somit ebenfalls sinnvoll genutzt werden. Vorzugsweise enthält das Futtermittel außerdem auch Schalen von Ölfrüchten, bevorzugter zerkleinerte Schalen von Ölfrüchten, insbesondere gemahlene Schalen von Ölfrüchten, wobei die Ölfrüchte vorzugsweise Sojabohnen, Sonnenblumenkerne und/oder Rapskörner enthalten, insbesondere wobei die Ölfrüchte Sojabohnen sind. Somit können auch die Schalen der Ölfrüchte einer sinnvollen Verwendung zugeführt werden.

### Kombinierte Anlage

Die Erfindung betrifft weiters eine kombinierte Anlage zur Herstellung von Bioethanol und einem Ölfruchtproteinprodukt mit dem erfindungsgemäßen, kombinierten Verfahren, aufweisend
*eine erste Anlage zur Herstellung des Bioethanols, aufweisend*
   einen Fermenter zum Fermentieren der Maische, und
   eine erste Trenneinheit zum Abtrennen zumindest eines Teils des Bioethanols von der fermentierten Brühe; und
*eine zweite Anlage zur Herstellung des Ölfruchtproteinprodukts, aufweisend*
   eine erste Extraktionsvorrichtung zum Extrahieren des Ölfruchtausgangsprodukts mit dem ersten Extraktionsmittel,
   eine zweite Trenneinheit zum Trennen des ersten Extraktionsprodukts in die erste Mischung und die zweite Mischung,
   eine dritte Trenneinheit zum Abtrennen zumindest eines Teils des Ölfruchtproteinprodukts von der ersten Mischung, und
   gegebenenfalls eine vierte Trenneinheit zum Abtrennen zumindest eines Teils des Ölfruchtnebenprodukts von der zweiten Mischung;
wobei die zweite Anlage weiters eine fünfte Trenneinheit zum Abtrennen zumindest eines Teils des in der ersten Miscella enthaltenen, ersten Extraktionsmittels aufweist.

Die erste Trenneinheit der ersten Anlage ist dem Fermenter nachgeschaltet. Vorzugsweise weist die erste Trenneinheit eine Destillationsapparatur auf. Hierdurch kann zumindest ein Teil des Bioethanols nicht nur von der fermentierten Brühe abgetrennt werden, sondern auch von zumindest einem Teil des Wassers, wodurch eine Bioethanol-Wasser-Mischung erhalten wird. Weiters weist die erste Trenneinheit vorzugsweise ein Molekularsieb, eine Membran und/oder ein Adsorbens auf, besonders bevorzugt ein Molekularsieb. Hierdurch kann die Bioethanol-Wasser-Mischung in Bioethanol und Wasser getrennt werden. Dabei ist das Molekularsieb, die Membran und/oder das Adsorbens der Destillationsapparatur der ersten Trenneinheit vorzugsweise nachgeschaltet. Vorzugsweise ist ein Abschnitt der ersten Trenneinheit, die das Molekularsieb aufweist, mit dem Fermenter derart verbunden, dass Kohlenstoffdioxid vom Fermenter zu diesem Abschnitt der ersten Trenneinheit geführt werden kann. Alternativ oder zusätzlich ist es bevorzugt, wenn dieser Abschnitt der ersten Trenneinheit, die das Molekularsieb aufweist, mit der Destillationsapparatur der ersten Trenneinheit derart verbunden ist, dass ein zur Reinigung des Molekularsiebs verwendetes, ethanolhaltiges Reinigungsmittel von dem Abschnitt der ersten Trenneinheit, die das Molekularsieb aufweist, zur Destillationsapparatur geführt werden kann. Dann kann das verwendete, ethanolhaltige Reinigungsmittel der fermentierten Brühe zugegeben werden, bevorzugt nach einem zumindest teilweisen Abtrennen von Wasser und/oder einer Schlempe von der fermentierten Brühe, insbesondere nach einem teilweisen Destillieren der fermentierten Brühe. Weiters ist es alternativ oder zusätzlich bevorzugt, dass der Abschnitt der ersten Trenneinheit, die das Molekularsieb aufweist, mit der zweiten Trenneinheit verbunden ist, insbesondere mit einer zweiten Extraktionsvorrichtung der zweiten Trenneinheit. Dann kann ethanolhaltiges Reinigungsmittel, das zur Entwässerung (Regenerierung) des Molekularsiebs verwendet wurde, der zweiten Extraktionsvorrichtung zur Verwendung als zweites Extraktionsmittel zugeführt werden.

Die erste Anlage weist vorzugsweise weiters eine Maischapparatur zum Maischen einer Mischung enthaltend das kohlenhydrathaltige Ausgangsmaterial und das Ölfruchtnebenprodukt auf. Diese ist dem Fermenter vorgeschaltet. Die Maischapparatur ist vorzugsweise mit der vierten Trenneinheit verbunden. Das im zweiten Verfahren anfallende Ölfruchtnebenprodukt kann dann unmittelbar von der vierten Trenneinheit der Maischapparatur zugeführt werden.

Die erste Anlage weist vorzugsweise weiters eine Zerkleinerungseinheit zur Zerkleinerung des kohlenhydrathaltigen Ausgangsmaterials, und gegebenenfalls von Schalen von Ölfrüchten, insbesondere Sojabohnen, auf. Die Zerkleinerungseinheit kann eine Zerkleinerungseinheit zum Trockenmahlen und/oder Nassmahlen sein. Die Zerkleinerungseinheit kann eine Mühle und/oder eine Stampfe aufweisen. Die Mühle kann z.B. ein Kegel-, Scheiben und/oder Schlagmahlwerk aufweisen. Hierdurch kann das kohlenhydrathaltige Ausgangsmaterial effizient zerkleinert werden. Die Zerkleinerungseinheit ist dem Fermenter, und gegebenenfalls der Maischapparatur, vorgeschaltet.

Weiters ist es bevorzugt, wenn die erste Anlage einen Trockner, insbesondere einen Futtermitteltrockner, zum Trocknen der Schlempe aufweist. Der Trockner ist vorzugsweise mit der ersten Trenneinheit verbunden, insbesondere mit der Destillationsapparatur der ersten Trenneinheit. Dann kann die in der ersten Trenneinheit, insbesondere in der Destillationsapparatur, abgetrennte Schlempe dem Trockner zugeführt werden. Wenn vorhanden, ist der Trockner vorzugsweise mit der Zerkleinerungseinheit der ersten Anlage verbunden, insbesondere, wenn die Zerkleinerungseinheit eingerichtet ist, das kohlenhydrathaltige Ausgangsmaterial im nassen Zustand zu zerkleinern. Dann können beim Zerkleinern, insbesondere beim Nassmahlen, vom kohlenhydrathaltigen Ausgangsmaterial abgetrennte Fasern, Maiskleber und/oder Maiskleberfutter gemeinsam mit der Schlempe getrocknet werden.

Die erste Anlage, insbesondere die erste Trenneinheit, weist weiters vorzugsweise einen ersten Sammelbehälter zum Sammeln des im ersten Verfahren hergestellten Bioethanols auf.

Bevorzugt ist es, wenn die erste Extraktionsvorrichtung mit der ersten Trenneinheit, insbesondere mit dem ersten Sammelbehälter, verbunden ist, insbesondere derart, dass im ersten Verfahren hergestelltes und gegebenenfalls im ersten Sammelbehälter gesammeltes Bioethanol der ersten Extraktionsvorrichtung zur Verwendung als erstes Extraktionsmittel zugeführt werden kann. Dies vereinfacht die Verfahrensführung, da in der ersten Anlage hergestelltes Bioethanol direkt der ersten Extraktionsvorrichtung zugeführt werden kann.

Die zweite Trenneinheit ist der ersten Extraktionsvorrichtung nachgeschaltet. Wenn das herzustellende Ölfruchtproteinprodukt ein Ölfruchtproteinkonzentrat ist, weist die zweite Trenneinheit vorzugsweise eine zweite Extraktionsvorrichtung zum Trennen des ersten Extraktionsprodukts in die erste Mischung und die zweite Mischung auf. Wenn das herzustellende Ölfruchtproteinprodukt ein Ölfruchtproteinisolat ist, weist die zweite Trenneinheit vorzugsweise einen Trockner und eine Fällungsapparatur auf. Dabei ist die Fällungsapparatur dem Trockner nachgeschaltet. Mit dem Trockner kann zumindest ein Teil des im ersten Extraktionsprodukt enthaltenen ersten Extraktionsmittels vom ersten Extraktionsprodukt abgetrennt werden. Mit der Fällungsapparatur kann das erste Extraktionsprodukt in die erste (ausgefällte) Mischung und in die zweite Mischung getrennt werden.

Die dritte Trenneinheit ist der zweiten Trenneinheit nachgeschaltet. Vorzugsweise weist die dritte Trenneinheit einen Trockner auf. Der Trockner kann eingerichtet sein, zumindest einen Teil des Ölfruchtproteinprodukts von der ersten Mischung unter reduziertem Druck (z.B. bei 800 mbar oder darunter) und/oder bei erhöhter Temperatur abzutrennen. Auch kann das abgetrennte Ölfruchtproteinprodukt im Trockner der dritten Trenneinheit noch nachgetrocknet werden, um etwaiges verbliebenes, zweites Extraktionsmittel zu entfernen.

Die vierte Trenneinheit ist mit der zweiten Trenneinheit verbunden. Sie ist zum Abtrennen zumindest eines Teils des Ölfruchtnebenprodukts von der zweiten Mischung eingerichtet. Die vierte Trenneinheit weist vorzugsweise einen Stripper, eine Destillationsapparatur, eine Zentrifuge, einen Dekanter, einen Sedimenter und/oder einen Verdampfer auf. Wenn das Ölfruchtnebenprodukt die Ölfruchtmelasse enthält, weist die vierte Trenneinheit vorzugsweise eine Destillationsapparatur oder einen Stripper auf, die bzw. der eingerichtet ist, sowohl einen Teil des Ölfruchtnebenprodukts als auch einen Teil des zweiten Extraktionsmittels von der zweiten Mischung abzutrennen. Das Abtrennen kann somit in derselben Destillationsapparatur bzw. im selben Stripper erfolgen, was den Aufbau der kombinierten Anlage sowie die Verfahrensführung maßgeblich vereinfacht. Vorzugsweise ist die vierte Trenneinheit mit der Maischapparatur und/oder mit dem Trockner, insbesondere Futtermitteltrockner, der ersten Anlage verbunden. Dann kann in der vierten Trenneinheit abgetrenntes Ölfruchtnebenprodukt der ersten Anlage zugeführt werden, sodass es in der in Schritt (a) bereitgestellten Maische enthalten sein kann, und/oder das Ölfruchtnebenprodukt kann dem Trockner zugeführt und gemeinsam mit der Schlempe getrocknet werden.

Die zweite Anlage weist vorzugsweise außerdem eine fünfte Trenneinheit zum Abtrennen zumindest eines Teils des in der ersten Miscella enthaltenen, ersten Extraktionsmittels und/oder zum Abtrennen zumindest eines Teils des in der ersten Miscella enthaltenen Öls und/oder Fetts auf. Die fünfte Trenneinheit ist vorzugsweise mit der ersten Extraktionsvorrichtung verbunden. Vorzugsweise weist die fünfte Trenneinheit eine Destillationsapparatur oder einen Stripper auf, die bzw. der eingerichtet ist, sowohl einen Teil des ersten Extraktionsmittels als auch einen Teil des Öls und/oder Fetts von der ersten Miscella abzutrennen. Das Abtrennen kann somit in derselben Destillationsapparatur bzw. im selben Stripper erfolgen, was den Aufbau der kombinierten Anlage sowie die Verfahrensführung maßgeblich vereinfacht. Wenn die zweite Trenneinheit eine zweite Extraktionsvorrichtung aufweist, ist die fünfte Trenneinheit vorzugsweise mit der zweiten Trenneinheit, insbesondere mit der zweiten Extraktionsvorrichtung, verbunden. Dann kann von der ersten Miscella abgetrenntes, erstes Extraktionsmittel der zweiten Trenneinheit zur Verwendung als zweites Extraktionsmittel zugeführt werden. Zusätzlich oder alternativ dazu ist es auch bevorzugt, wenn die fünfte Trenneinheit mit der ersten Trenneinheit, insbesondere mit der Destillationsapparatur der ersten Trenneinheit, verbunden ist. Dann kann von der ersten Miscella abgetrenntes, erstes Extraktionsmittel der ersten Trenneinheit, insbesondere der Destillationsapparatur zugeführt werden, und der in Schritt (b) des ersten Verfahrens erhaltenen, fermentierten Brühe zugegeben werden, bevorzugt nach einem zumindest teilweisen Abtrennen von Wasser und/oder einer Schlempe von der fermentierten Brühe, insbesondere nach einem teilweisen Destillieren der fermentierten Brühe.

Die zweite Anlage weist vorzugsweise weiters einen Verdampfer auf, der zum Eindampfen der zweiten Mischung und/oder des Ölfruchtnebenprodukts eingerichtet ist. Dieser Verdampfer ist vorzugsweise mit der zweiten Trenneinheit und/oder mit der vierten Trenneinheit verbunden, sowie mit der Maischapparatur, wenn vorhanden. Somit kann die in Schritt (iii) des kombinierten Verfahrens erhaltene zweite Mischung, insbesondere zweite Miscella, und/oder das Ölfruchtnebenprodukt vor der Verwendung im ersten Verfahren eingedampft werden.

Bevorzugt ist es, wenn die zweite Anlage eine Aufbereitungseinheit zum Aufbereiten des bereitgestellten Ölfruchtausgangsprodukts aufweist. Die Aufbereitungseinheit ist der ersten Extraktionsvorrichtung vorgeschaltet. Vorzugsweise weist die Aufbereitungseinheit eine Schäleinheit zum Schälen des Ölfruchtausgangsprodukts und/oder eine Zerkleinerungseinheit zum Zerkleinern des Ölfruchtausgangsprodukts auf. Die Zerkleinerungseinheit kann eine Mühle und/oder eine Stampfe aufweisen. Die Mühle kann z.B. ein Kegel-, Scheiben und/oder Schlagmahlwerk aufweisen. Hierdurch kann das Ölfruchtausgangsprodukt effizient zerkleinert werden. Die Aufbereitungseinheit, insbesondere die Schäleinheit, der zweiten Anlage ist vorzugsweise mit der Zerkleinerungseinheit der ersten Anlage verbunden. Dann können von den Ölfrüchten abgetrennte Schalen der Zerkleinerungseinheit der ersten Anlage zugeführt und gemeinsam mit dem kohlenhydrathaltigen Ausgangsmaterial zerkleinert werden. Alternativ oder zusätzlich ist die Aufbereitungseinheit, insbesondere die Schäleinheit, der zweiten Anlage bevorzugt mit dem Trockner, insbesondere Futtermitteltrockner, der ersten Anlage verbunden. Dann können von den Ölfrüchten, insbesondere Sojabohnen, abgetrennte Schalen gemeinsam mit der Schlempe getrocknet werden.

Die zweite Anlage, insbesondere die dritte Trenneinheit, weist weiters vorzugsweise einen zweiten Sammelbehälter zum Sammeln des im zweiten Verfahren hergestellten Ölfruchtproteinprodukts auf.

Vorzugsweise weist die kombinierte Anlage, insbesondere die erste Trenneinheit, einen Lagerbehälter zur Lagerung von abgetrenntem ersten Extraktionsmittel, abgetrenntem zweiten Extraktionsmittel und/oder abgetrenntem Reinigungsmittel, jeweils enthaltend Ethanol, vorzugsweise Bioethanol, auf. Dieser Lagerbehälter ist vorzugsweise mit der Destillationsapparatur der ersten Trenneinheit, verbunden. Weiters ist der Lagerbehälter vorzugsweise mit dem Abschnitt der ersten Trenneinheit, die das Molekularsieb aufweist, mit der zweiten Trenneinheit, mit der dritten Trenneinheit, mit der vierten Trenneinheit und/oder mit der fünften Trenneinheit verbunden. Im Lagerbehälter können zur Reinigung des Molekularsiebs verwendetes, ethanolhaltiges Reinigungsmittel; von der ersten Miscella abgetrenntes, erstes Extraktionsmittel; von der zweiten Mischung abgetrenntes, zweites Extraktionsmittel; und/oder vom Ölfruchtproteinprodukt, insbesondere Ölfruchtproteinkonzentrat, abgetrenntes, zweites Extraktionsmittel gelagert werden, insbesondere vor deren Weiterverwendung im kombinierten Verfahren.

Vorzugsweise weist die kombinierte Anlage, insbesondere die erste Anlage, eine Abwasserbehandlungseinheit auf. Die Abwasserbehandlungseinheit ist vorzugsweise mit der ersten Trenneinheit und/oder mit dem Trockner (insbesondere Futtermitteltrockner) der ersten Anlage verbunden. Dann kann in diesen Anlagenkomponenten abgetrenntes Wasser der Abwasserbehandlungseinheit zur Reinigung zugeführt werden.

Die erste Extraktionsvorrichtung, die zweite Trenneinheit, die dritte Trenneinheit, die vierte Trenneinheit, die fünfte Trenneinheit und/oder die Aufbereitungsanlage ist/sind vorzugsweise mit der ersten Trenneinheit, insbesondere mit dem Molekularsieb, und/oder mit dem Trockner, insbesondere Futtermitteltrockner, der ersten Anlage verbunden, insbesondere mit einem zum Transport von thermischer Energie geeigneten Verbindungselement. Das Verbindungselement kann ein Rohr und/oder eine Leitung aufweisen, vorzugsweise mit einer thermischen Isolierung. In der kombinierten Anlage, insbesondere in der ersten Anlage, gewonnene thermische Energie kann dann im kombinierten Verfahren, insbesondere in der zweiten Anlage, verwendet werden. Die thermische Energie kann durch bekannte Verfahren transportiert werden. Beispielsweise kann die thermische Energie mittels eines Wärmeträgerfluids, insbesondere Wasser, transportiert werden.

In einer bevorzugten Ausführungsform weist die zweite Anlage zwei zweite Trenneinheiten, zwei dritte Trenneinheiten, gegebenenfalls zwei vierte Trenneinheiten, und gegebenenfalls zwei zweite Sammelbehälter auf. Die zweite Anlage ist dann eingerichtet, Ölfruchtproteinisolat und Ölfruchtproteinkonzentrat gleichzeitig und/oder hintereinander herzustellen. So kann etwa ein erster Teil des ersten Extraktionsprodukts zur Herstellung des Ölfruchtproteinkonzentrats, insbesondere Sojaproteinkonzentrats, und ein zweiter Teil des ersten Extraktionsprodukts zur Herstellung des Ölfruchtproteinisolats, insbesondere Sojaproteinisolats, verwendet werden.

Wenn in der vorliegenden Offenbarung von "Ethanol" die Rede ist, ist "Bioethanol" mitgemeint; das gilt nicht für den umgekehrten Fall, d.h. wenn von "Bioethanol" die Rede ist, ist "Ethanol" (welches z.B. auch aus Erdöl gewonnen werden kann) nicht mitgemeint.

Damit, dass eine Anlagenkomponente mit einer weiteren verbunden ist, ist in dieser Offenbarung gemeint, dass diese beiden Anlagenkomponenten in Fluidverbindung stehen. Mittels der Fluidverbindung kann ein Fluid (z.B. Bioethanol) unmittelbar zwischen Anlagenkomponenten transportiert werden, was die Verfahrensführung vereinfacht.

### Erfindungsgemäß erhältliche Ölfruchtproteinprodukte

Gemäß einem weiteren Aspekt betrifft die vorliegende Erfindung Ölfruchtproteinprodukte, die mit dem erfindungsgemäßen kombinierten Verfahren erhältlich sind, insbesondere Ölfruchtproteinkonzentrate, Ölfruchtproteinisolate, Ölfruchtöl-Produkte, Ölfrucht-Lecithin-Produkte oder Ölfrucht-Faserprodukte. Besonders bevorzugt sind dabei Produkte, die auf der Verwendung von Soja-basierten Ausgangsmaterialien erhältlich sind, also insbesondere Sojaproteinkonzentrate (SPCs), Sojaproteinisolate (SPIs), Sojaöl-Produkte, Soja-Lecithin-Produkte oder Soja-Faserprodukte.

Die mit dem erfindungsgemäßen Kombinationsverfahren hergestellten Ölfruchtproteinprodukte weisen dabei aufgrund der Extraktion mit dem Bioethanol aus dem ersten Verfahren Besonderheiten auf, die sich positiv im Hinblick auf Gehalt und Wirksamkeit der Inhaltsstoffe, wie z.B. Phytoöstrogene oder Inhibitoren (Trypsininhibitoren), oder im Hinblick auf bekannte Verunreinigungen im Ausgangsmaterial (Tropanalkaloiden (aus Bilsenkraut, Stechapfel oder Tollkirsche, wie z.B. Atropin und Scopolamin) oder Mykotoxinen (Deoxynivalenol, T2- und HT2-Toxine oder Zearalenon)) im Endprodukt auswirken. Vor allem können sich in einem stand-alone Ethanolverfahren derartige Verunreinigungen wie Mykotoxine anreichern, weil dabei Ethanol ständig zirkuliert wird. Diese Anreicherung wird durch den erfindungsgemäßen Drain im Hauptprodukt des Kombinationsverfahrens beim Ethanolverfahren verhindert oder zumindest verringert und ist somit jedenfalls von Vorteil. Besonders die Qualität des verwendeten Bioethanols aus dem ersten Verfahren als Extraktionsmittel erlaubt eine schonende und verbesserte Gewinnung der Ölfruchtproteinprodukte im erfindungsgemäßen Verfahren, wodurch auch insgesamt verbesserte Ölfruchtproteinprodukte erhalten werden können.

Die Erfindung betrifft insbesondere die folgenden Ausführungsformen:
1. Kombiniertes Verfahren, umfassend ein erstes Verfahren zur Herstellung von Bioethanol und ein zweites Verfahren zur Herstellung eines Ölfruchtproteinprodukts,
   *das erste Verfahren umfassend die Schritte*
      (a) Bereitstellen einer Maische, wobei die Maische ein kohlenhydrathaltiges Ausgangsmaterial enthält,
      (b) Fermentieren der Maische, um eine fermentierte Brühe enthaltend Bioethanol zu erhalten, und
      (c) Abtrennen zumindest eines Teils des Bioethanols von der fermentierten Brühe; und
   *das zweite Verfahren umfassend die Schritte*
      (i) Bereitstellen eines Ölfruchtausgangsprodukts,
      (ii) Extrahieren des Ölfruchtausgangsprodukts mit einem ersten Extraktionsmittel, um eine erste Miscella und ein erstes Extraktionsprodukt zu erhalten,
      (iii) Trennen des ersten Extraktionsprodukts in eine erste Mischung enthaltend das Ölfruchtproteinprodukt und in eine zweite Mischung enthaltend das Ölfruchtnebenprodukt,
      (iv) Abtrennen zumindest eines Teils des Ölfruchtproteinprodukts von der ersten Mischung, und gegebenenfalls Abtrennen zumindest eines Teils des Ölfruchtnebenprodukts von der zweiten Mischung;
   vorzugsweise wobei die in Schritt (a) des ersten Verfahrens bereitgestellte Maische weiters zumindest einen Teil des im zweiten Verfahren erhaltenen Ölfruchtnebenprodukts enthält.
2. Kombiniertes Verfahren, umfassend ein erstes Verfahren zur Herstellung von Bioethanol und ein zweites Verfahren zur Herstellung eines Ölfruchtproteinprodukts,
   *das erste Verfahren umfassend die Schritte*
      (a) Bereitstellen einer Maische, wobei die Maische ein kohlenhydrathaltiges Ausgangsmaterial enthält,
      (b) Fermentieren der Maische, um eine fermentierte Brühe enthaltend Bioethanol zu erhalten, und
      (c) Abtrennen zumindest eines Teils des Bioethanols von der fermentierten Brühe; und
   *das zweite Verfahren umfassend die Schritte*
      (i) Bereitstellen eines Ölfruchtausgangsprodukts,
      (ii) Extrahieren des Ölfruchtausgangsprodukts mit einem ersten Extraktionsmittel, um eine erste Miscella und ein erstes Extraktionsprodukt zu erhalten,
      (iii) Trennen des ersten Extraktionsprodukts in eine erste Mischung enthaltend das Ölfruchtproteinprodukt und in eine zweite Mischung enthaltend das Ölfruchtnebenprodukt,
      (iv) Abtrennen zumindest eines Teils des Ölfruchtproteinprodukts von der ersten Mischung, und gegebenenfalls Abtrennen zumindest eines Teils des Ölfruchtnebenprodukts von der zweiten Mischung;
   wobei die in Schritt (a) des ersten Verfahrens bereitgestellte Maische weiters zumindest einen Teil des im zweiten Verfahren erhaltenen Ölfruchtnebenprodukts enthält oder zumindest ein Teil des im zweiten Verfahren erhaltenen Ölfruchtnebenprodukts wahrend Schritt (b) zugesetzt wird,
   wobei das erste Extraktionsmittel im ersten Verfahren hergestelltes Bioethanol enthält, vorzugweise wobei das erste Extraktionsmittel im ersten Verfahren hergestelltes Bioethanol ist,
   wobei die erste Miscella zumindest einen Teil des ersten Extraktionsmittels enthält, und
   wobei zumindest ein Teil des in der ersten Miscella enthaltenen, ersten Extraktionsmittels von der ersten Miscella abgetrennt und in Schritt (c) des ersten Verfahrens verwendet wird, vorzugsweise wobei das abgetrennte, erste Extraktionsmittel der in Schritt (b) des ersten Verfahrens erhaltenen, fermentierten Brühe zugegeben wird, insbesondere nach einem zumindest teilweisen Abtrennen von Wasser und/oder einer Schlempe von der fermentierten Brühe.
3. Kombiniertes Verfahren, umfassend ein erstes Verfahren zur Herstellung von Bioethanol und ein zweites Verfahren zur Herstellung eines Ölfruchtproteinprodukts,
   *das erste Verfahren umfassend die Schritte*
      (a) Bereitstellen einer Maische, wobei die Maische ein kohlenhydrathaltiges Ausgangsmaterial enthält,
      (b) Fermentieren der Maische, um eine fermentierte Brühe enthaltend Bioethanol zu erhalten, und
      (c) Abtrennen zumindest eines Teils des Bioethanols von der fermentierten Brühe; und
   *das zweite Verfahren umfassend die Schritte*
      (i) Bereitstellen eines Ölfruchtausgangsprodukts,
      (ii) Extrahieren des Ölfruchtausgangsprodukts mit einem ersten Extraktionsmittel, um eine erste Miscella und ein erstes Extraktionsprodukt zu erhalten,
      (iii) Trennen des ersten Extraktionsprodukts in eine erste Mischung enthaltend das Ölfruchtproteinprodukt und in eine zweite Mischung enthaltend das Ölfruchtnebenprodukt,
      (iv) Abtrennen zumindest eines Teils des Ölfruchtproteinprodukts von der ersten Mischung, und gegebenenfalls Abtrennen zumindest eines Teils des Ölfruchtnebenprodukts von der zweiten Mischung;
   wobei das Bereitstellen des Ölfruchtausgangsprodukts in Schritt (i) des zweiten Verfahrens ein Abtrennen von Schalen von Ölfrüchten umfasst, und
   wobei die in Schritt (a) des ersten Verfahrens bereitgestellte Maische weiters zumindest einen Teil des im zweiten Verfahren erhaltenen Ölfruchtnebenprodukts und zerkleinerte Schalen von Ölfrüchten enthält, wobei die Schalen zumindest ein Teil der in Schritt (i) des zweiten Verfahrens von den Ölfrüchten abgetrennten Schalen sind.
4. Kombiniertes Verfahren nach einer der Ausführungsformen 1 bis 3, wobei die in Schritt (a) bereitgestellte Maische durch Maischen des kohlenhydrathaltigen Ausgangsmaterials und des Ölfruchtnebenprodukts, vorzugsweise in Anwesenheit von Wasser, erhalten wird.
5. Kombiniertes Verfahren nach einer der Ausführungsformen 1 bis 4, wobei das kohlenhydrathaltige Ausgangsmaterial ein Getreide ist, vorzugsweise Mais und/oder Weizen.
6. Kombiniertes Verfahren nach einer der Ausführungsformen 1 bis 5, wobei das Maischen bei einer Temperatur von 60 bis 130 °C durchgeführt wird, vorzugsweise bei 80 bis 120 °C; und/oder wobei das Maischen für eine Zeitdauer von 0,5 h oder darüber erfolgt, vorzugsweise für 2 h oder darüber, bevorzugt für 1 bis 4 h.
7. Kombiniertes Verfahren nach einer der Ausführungsformen 1 bis 6, wobei das Maischen in Anwesenheit eines Enzyms erfolgt, wobei das Enzym vorzugsweise ausgewählt ist aus der Gruppe umfassend eine Alpha-Amylase, eine Glucanase, eine Glucoamylase, eine Cellulase, eine Hemicellulase, eine Beta-Glucanase, eine Xylanase, eine Arabinase, eine Galactosidase und eine Protease, oder eine Mischung davon, Enzym besonders bevorzugt eine Alpha-Amylase und/oder eine Glucoamylase.
8. Kombiniertes Verfahren nach einer der Ausführungsformen 1 bis 5, wobei das kohlenhydrathaltige Ausgangsmaterial vor dem Maischen zerkleinert wird, vorzugsweise mittels Trockenmahlen und/oder Nassmahlen.
9. Kombiniertes Verfahren nach Ausführungsform 8, wobei das kohlenhydrathaltige Ausgangsmaterial vor dem Maischen mittels Trockenmahlen zerkleinert wird; und/oder wobei das kohlenhydrathaltige Ausgangsmaterial vor dem Maischen mittels Nassmahlen in Gegenwart von Wasser zerkleinert wird.
10. Kombiniertes Verfahren nach Ausführungsform 8 oder 9, wobei das kohlenhydrathaltige Ausgangsmaterial und Schalen von Ölfrüchten vor dem Maischen gemeinsam zerkleinert werden, vorzugsweise mittels Trockenmahlen; vorzugsweise wobei die Schalen der Ölfrüchte zumindest ein Teil von in Schritt (i) des zweiten Verfahrens von Ölfrüchten abgetrennten Schalen sind.
11. Kombiniertes Verfahren nach einer der Ausführungsformen 8 bis 10, wobei vor dem Maischen zumindest ein Teil von beim Zerkleinern, insbesondere beim Nassmahlen, des kohlenhydrathaltigen Ausgangsmaterials anfallenden Fasern, Maiskleber und/oder Maiskleberfutter vom zerkleinerten, kohlenhydrathaltigen Ausgangsmaterial abgetrennt wird; vorzugsweise wobei die abgetrennten Fasern, der Maiskleber und/oder das Maiskleberfutter einer von der fermentierten Brühe abgetrennten Schlempe zugegeben werden.
12. Kombiniertes Verfahren nach einer der Ausführungsformen 1 bis 11, wobei das Ölfruchtnebenprodukt, das in der in Schritt (a) bereitgestellten Maische enthalten ist, zumindest einen Teil der in Schritt (iii) des zweiten Verfahrens erhaltenen zweiten Mischung und/oder zumindest einen Teil des in Schritt (iv) erhaltenen Ölfruchtnebenprodukts enthält.
13. Kombiniertes Verfahren nach einer der Ausführungsformen 1 bis 12, wobei das Ölfruchtnebenprodukt eine Ölfruchtmelasse und/oder einen Ölfruchtproteinliquor enthält.
14. Kombiniertes Verfahren nach einer der Ausführungsformen 1 bis 13, wobei das Ölfruchtnebenprodukt ein Trockensubstanzgewicht von 10 bis 80 Gew% aufweist, bevorzugter von 15 bis 50 Gew%, noch bevorzugter von 20 bis 40 Gew%, besonders bevorzugt von 25 bis 35 Gew%, bezogen auf das Gesamtgewicht des Ölfruchtnebenprodukts.
15. Kombiniertes Verfahren nach einer der Ausführungsformen 1 bis 14, wobei die in Schritt (a) bereitgestellte Maische das Ölfruchtnebenprodukt in einer Menge von 30 Gew% oder darunter enthält, bevorzugter 20 Gew% oder darunter, noch bevorzugter 15 Gew% oder darunter, noch bevorzugter 12 Gew% oder darunter, noch bevorzugter 10 Gew% oder darunter, bezogen auf das Gewicht des kohlenhydrathaltigen Ausgangsmaterials.
16. Kombiniertes Verfahren nach Ausführungsform 15, wobei die in Schritt (a) bereitgestellte Maische das Ölfruchtnebenprodukt in einer Menge von 1 bis 30 Gew% enthält, vorzugsweise 1 bis 20 Gew%, bevorzugter 1 bis 15 Gew%, noch bevorzugter 1 bis 12 Gew%, besonders bevorzugt 5 bis 10 Gew%, bezogen auf das Gewicht des kohlenhydrathaltigen Ausgangsmaterials.
17. Kombiniertes Verfahren nach Ausführungsform 16, wobei die Maische ein Verhältnis von Ölfruchtnebenprodukt zu kohlenhydrathaltigem Ausgangsmaterial von 0,1:10 bis 1,5:10 aufweist (Gew%/Gew%).
18. Kombiniertes Verfahren nach einer der Ausführungsformen 1 bis 17, wobei die in Schritt (a) bereitgestellte Maische Schalen von Ölfrüchten enthält, vorzugsweise zerkleinerte Schalen von Ölfrüchten, wobei die Schalen vorzugsweise zumindest ein Teil von in Schritt (i) des zweiten Verfahrens von Ölfrüchten abgetrennten Schalen sind.
19. Kombiniertes Verfahren nach einer der Ausführungsformen 1 bis 18, wobei der Maische vor und/oder in Schritt (b) ein Mikroorganismus zugegeben wird, insbesondere eine Hefe und/oder ein Bakterium.
20. Kombiniertes Verfahren nach einer der Ausführungsformen 1 bis 19, wobei die Maische in Schritt (b) bei einer Temperatur von 20 bis 40 °C fermentiert wird, bevorzugter von 28 bis 36 °C.
21. Kombiniertes Verfahren nach einer der Ausführungsformen 1 bis 20, wobei die Maische in Schritt (b) für eine Zeitdauer von 36 h oder darüber fermentiert wird, bevorzugter für 55 h oder darüber, besonders bevorzugt für eine Zeitdauer von 50 bis 70 h.
22. Kombiniertes Verfahren nach einer der Ausführungsformen 1 bis 21, wobei die in Schritt (b) erhaltene, fermentierte Brühe Bioethanol und eine Schlempe enthält, wobei die Schlempe vorzugsweise ein Protein, Biomasse des Mikroorganismus, ein Fett und/oder einen Mineralstoff enthält; vorzugsweise wobei in Schritt (c) des ersten Verfahrens zumindest ein Teil der in der fermentierten Brühe enthaltenen Schlempe von der fermentierten Brühe abgetrennt wird; besonders bevorzugt wobei die abgetrennte Schlempe nachfolgend getrocknet wird.
23. Kombiniertes Verfahren nach Ausführungsform 22, wobei das Abtrennen zumindest eines Teils des Bioethanols von der fermentierten Brühe in Schritt (c), und gegebenenfalls das Abtrennen zumindest eines Teils der Schlempe von der fermentierten Brühe in Schritt (c), ein thermisches Abtrennen, insbesondere ein Destillieren, umfasst.
24. Kombiniertes Verfahren nach Ausführungsform 23, wobei durch das Destillieren die Schlempe von einer Bioethanol-Wasser-Mischung abgetrennt werden kann, wobei die Bioethanol-Wasser-Mischung vorzugsweise einen Anteil von Bioethanol von 70 Gew% oder darüber enthält, bevorzugter 75 Gew% oder darüber, noch bevorzugter 80 Gew% oder darüber, insbesondere 90 bis 95,6 Gew%, bezogen auf das Gewicht der Bioethanol-Wasser-Mischung.
25. Kombiniertes Verfahren nach Ausführungsform 24, wobei das Abtrennen zumindest eines Teils des Bioethanols von der fermentierten Brühe in Schritt (c) das Destillieren und ein nachfolgendes Trennen der Bioethanol-Wasser-Mischung in Bioethanol und Wasser umfasst, wobei das Trennen der Bioethanol-Wasser-Mischung in Bioethanol und Wasser vorzugsweise mittels eines Molekularsiebs, eines Membranverfahrens und/oder einer Druckwechseladsorption erfolgt.
26. Kombiniertes Verfahren nach Ausführungsform 25, wobei die Bioethanol-Wasser-Mischung mittels eines Molekularsiebs in Bioethanol und Wasser getrennt werden, wobei die Temperatur beim Trennen mittels des Molekularsiebs vorzugsweise im Bereich von 120 bis 180 °C liegt, bevorzugter von 160 bis 170 °C, und/oder wobei der Druck vorzugsweise im Bereich von 2 bis 6 bar liegt, bevorzugter von 3 bis 5 bar.
27. Kombiniertes Verfahren nach Ausführungsform 26, wobei die Reinigung des Molekularsiebs mit einem ethanolhaltigen Reinigungsmittel erfolgt, vorzugsweise wobei das Reinigungsmittel Ethanol in einer Menge von 70 Gew% oder darüber enthält, bevorzugter 80 Gew% oder darüber, noch bevorzugter 90 Gew% oder darüber, besonders bevorzugt 100 Gew%, bezogen auf das Gewicht des Reinigungsmittels; wobei das Ethanol besonders bevorzugt im ersten Verfahren hergestelltes Bioethanol ist.
28. Kombiniertes Verfahren nach Ausführungsform 27, wobei das ethanolhaltige Reinigungsmittel, insbesondere das bioethanolhaltige Reinigungsmittel, nach seiner Verwendung zur Reinigung des Molekularsiebs in Schritt (c) des ersten Verfahrens verwendet wird und/oder als zweites Extraktionsmittel verwendet wird.
29. Kombiniertes Verfahren nach einer der Ausführungsformen 26 bis 28, wobei die Reinigung des Molekularsiebs mit Kohlenstoffdioxid erfolgt, vorzugsweise wobei beim Fermentieren in Schritt (b) entstandenes Kohlenstoffdioxid verwendet wird.
30. Kombiniertes Verfahren nach einer der Ausführungsformen 1 bis 29, wobei das Abtrennen zumindest eines Teils des Bioethanols von der fermentierten Brühe in Schritt (c) ein Kondensieren von gasförmigem Bioethanol und/oder gasförmigem Wasser umfasst, vorzugsweise wobei dabei gewonnene thermische Energie im zweiten Verfahren genutzt wird, insbesondere für ein Zerkleinern von Ölfrüchten, um das in Schritt (i) bereitgestellte Ölfruchtausgangsprodukt zu erhalten, für das Extrahieren des Ölfruchtausgangsprodukts mit dem ersten Extraktionsmittel in Schritt (ii) und/oder für das Trennen des ersten Extraktionsprodukts in die erste und zweite Mischung in Schritt (iii).
31. Kombiniertes Verfahren nach einer der Ausführungsformen 22 bis 30, wobei in Schritt (c) zumindest ein Teil einer in der fermentierten Brühe enthaltenen Schlempe abgetrennt wird; vorzugsweise wobei die abgetrennte Schlempe nachfolgend getrocknet wird.
32. Kombiniertes Verfahren nach Ausführungsform 31, wobei die abgetrennte Schlempe für 0,1 h oder mehr getrocknet wird, bevorzugter für 0,15 h oder mehr, besonders bevorzugt für 0,15 bis 0,3 h; und/oder wobei das Trocknen der abgetrennten Schlempe vorzugsweise bei einer Temperatur von 60 °C oder darüber erfolgt, bevorzugter von 100 °C oder darüber, besonders bevorzugt bei 100 bis 140 °C.
33. Kombiniertes Verfahren nach Ausführungsform 31 oder 32, wobei beim Trocknen der Schlempe abgetrenntes, gasförmiges Wasser kondensiert wird, wobei thermische Energie gewonnen wird, die vorzugsweise im kombinierten Verfahren genutzt wird, insbesondere für ein Zerkleinern von Ölfrüchten, um das in Schritt (i) bereitgestellte Ölfruchtausgangsprodukt zu erhalten, für das Extrahieren des Ölfruchtausgangsprodukts mit dem ersten Extraktionsmittel in Schritt (ii) und/oder für das Trennen des ersten Extraktionsprodukts in die erste und zweite Mischung in Schritt (iii).
34. Kombiniertes Verfahren nach einer der Ausführungsformen 31 bis 33, wobei die abgetrennte Schlempe gemeinsam mit Schalen von Ölfrüchten getrocknet wird, vorzugsweise mit zerkleinerten Schalen von Ölfrüchten.
35. Kombiniertes Verfahren nach Ausführungsform 34, wobei der abgetrennten Schlempe ein Teil der in Schritt (i) des zweiten Verfahrens von den Ölfrüchten abgetrennten Schalen zugegeben wird.
36. Kombiniertes Verfahren nach einer der Ausführungsformen 31 bis 35, wobei der abgetrennten Schlempe ein Teil des im zweiten Verfahren erhaltenen Ölfruchtnebenprodukts zugegeben wird, vorzugsweise wobei die Schlempe und das zugegebene Ölfruchtnebenprodukt gemeinsam getrocknet werden.
37. Kombiniertes Verfahren nach einer der Ausführungsformen 31 bis 36, wobei die abgetrennte, vorzugsweise getrocknete, Schlempe als Futtermittel verwendet und/oder energetisch verwertet wird.
38. Kombiniertes Verfahren nach einer der Ausführungsformen 1 bis 37, wobei in Schritt (c) vom Bioethanol abgetrenntes Wasser und/oder beim Trocknen der Schlempe abgetrenntes Wasser aufbereitet wird.
39. Kombiniertes Verfahren nach einer der Ausführungsformen 1 bis 38, wobei das Ölfruchtproteinprodukt ein Ölfruchtproteinkonzentrat und/oder ein Ölfruchtproteinisolat ist; vorzugsweise wobei im zweiten Verfahren das Ölfruchtproteinisolat und das Ölfruchtproteinkonzentrat hergestellt werden, wobei ein erster Teil des ersten Extraktionsprodukts zur Herstellung des Ölfruchtproteinkonzentrats und ein zweiter Teil des ersten Extraktionsprodukts zur Herstellung des Ölfruchtproteinisolats verwendet werden.
40. Kombiniertes Verfahren nach einer der Ausführungsformen 1 bis 39, wobei das im zweiten Verfahren anfallende Ölfruchtnebenprodukt ein Kohlenhydrat (bzw. eine Mischung von zwei oder mehr Kohlenhydraten) enthält, wobei vorzugsweise zumindest ein Kohlenhydrat, insbesondere zumindest eine Stärke, durch Maischen verzuckerbar ist.
41. Kombiniertes Verfahren nach Ausführungsform 40, wobei das Ölfruchtnebenprodukt zumindest 15 Gew% eines verzuckerbaren Kohlenhydrats enthält, bevorzugter zumindest 20 Gew%, noch bevorzugter zumindest 25 Gew%, besonders bevorzugt zumindest 30 Gew%, bezogen auf den Anteil an Kohlenhydraten im Ölfruchtnebenprodukt, und bezogen auf das Trockensubstanzgewicht des Ölfruchtnebenprodukts.
42. Kombiniertes Verfahren nach einer der Ausführungsformen 1 bis 41, wobei das im zweiten Verfahren anfallende Ölfruchtnebenprodukt ein Protein (bzw. eine Mischung von zwei oder mehr Proteinen) enthält.
43. Kombiniertes Verfahren nach einer der Ausführungsformen 1 bis 42, wobei das Bereitstellen des Ölfruchtausgangsprodukts in Schritt (i) ein Abtrennen von Schalen von Ölfrüchten und/oder ein Zerkleinern der Ölfrüchte umfasst, vorzugsweise wobei das Ölfruchtausgangsprodukt geschälte und/oder zerkleinerte Sojabohnen, Sonnenblumenkörner und/oder Rapskörner enthält.
44. Kombiniertes Verfahren nach einer der Ausführungsformen 1 bis 43, wobei das Ölfruchtausgangsprodukt aus Ölfrüchten erhalten wird, wobei die Ölfrüchte Sojabohnen, Sonnenblumenkörner und/oder Rapskörner sind.
45. Kombiniertes Verfahren nach Ausführungsform 43 oder 44, wobei zumindest ein Teil der abgetrennten Schalen bei der Herstellung der in Schritt (a) des ersten Verfahrens bereitgestellten Maische verwendet und/oder gemeinsam mit der Schlempe getrocknet wird.
46. Kombiniertes Verfahren nach Ausführungsform 44 oder 45, wobei das Zerkleinern der Ölfrüchte bei erhöhter Temperatur erfolgt, bevorzugt bei einer Temperatur von 50 °C oder darüber erfolgt, bevorzugter von 70 °C oder darüber, noch bevorzugter von 90 °C oder darüber, besonders bevorzugt von 110 °C oder darüber.
47. Kombiniertes Verfahren nach Ausführungsform 46, wobei die für das Zerkleinern der Ölfrüchte bei erhöhter Temperatur benötigte thermische Energie zumindest zum Teil im ersten Verfahren gewonnene thermische Energie ist, insbesondere beim Abtrennen zumindest eines Teils des Bioethanols von der fermentierten Brühe in Schritt (c) gewonnene thermische Energie, und/oder beim Kondensieren des beim Trocknen der Schlempe abgetrennten Wassers gewonnene thermische Energie.
48. Kombiniertes Verfahren nach einer der Ausführungsformen 1 bis 47, wobei das in Schritt (ii) erhaltene, erste Extraktionsprodukt einen zumindest teilweise entfetteten und/oder entölten Feststoff enthält.
49. Kombiniertes Verfahren nach einer der Ausführungsformen 1 bis 48, wobei das Extrahieren in Schritt (ii) bei erhöhter Temperatur durchgeführt wird, vorzugweise bei 50°C oder darüber, bevorzugter bei 60°C oder darüber, noch bevorzugter bei 65°C bis 100°C, insbesondere bei 70 bis 90°C.
50. Kombiniertes Verfahren nach Ausführungsform 49, wobei die für das Extrahieren in Schritt (ii) bei erhöhter Temperatur benötigte thermische Energie zumindest zum Teil im kombinierten Verfahren gewonnene thermische Energie ist, bevorzugt beim Abtrennen zumindest eines Teils des Bioethanols von der fermentierten Brühe in Schritt (c) gewonnene thermische Energie, und/oder beim Kondensieren des beim Trocknen der Schlempe abgetrennten Wassers gewonnene thermische Energie.
51. Kombiniertes Verfahren nach einer der Ausführungsformen 1 bis 50, wobei das erste Extraktionsmittel Ethanol ist, wobei das Ethanol vorzugsweise eine Reinheit von 99,0 Gew% oder darüber aufweist, bevorzugter von 99,5 Gew% oder darüber, besonders bevorzugt von 99,9 Gew% oder darüber, bezogen auf das Gewicht des ersten Extraktionsmittels.
52. Kombiniertes Verfahren nach Ausführungsform 51, wobei das erste Extraktionsmittel im ersten Verfahren hergestelltes Bioethanol enthält, vorzugsweise wobei das erste Extraktionsmittel im ersten Verfahren hergestelltes Bioethanol ist.
53. Kombiniertes Verfahren nach einer der Ausführungsformen 1 bis 52, wobei zumindest ein Teil eines in der ersten Miscella enthaltenen Öls und/oder Fetts von der ersten Miscella abgetrennt wird, wobei das Abtrennen vorzugsweise ein Destillieren, insbesondere ein fraktioniertes Destillieren, und/oder Strippen umfasst.
54. Kombiniertes Verfahren nach einer der Ausführungsformen 1 bis 53, wobei zumindest ein Teil des in der ersten Miscella enthaltenen, ersten Extraktionsmittels von der ersten Miscella abgetrennt wird, vorzugsweise durch Destillieren und/oder Strippen.
55. Kombiniertes Verfahren nach Ausführungsform 54, wobei das von der ersten Miscella abgetrennte, erste Extraktionsmittel einen Ethanolgehalt von 85 Gew% oder darüber aufweist, bevorzugter von 90 Gew% oder darüber, noch bevorzugter von 95 Gew% oder darüber, bezogen auf das Gewicht des abgetrennten, ersten Extraktionsmittels.
56. Kombiniertes Verfahren nach Ausführungsform 54 oder 55, wobei das von der ersten Miscella abgetrennte, erste Extraktionsmittel (enthaltend Ethanol, insbesondere Bioethanol) der in Schritt (b) des ersten Verfahrens erhaltenen, fermentierten Brühe zugegeben wird; und/oder wobei das von der ersten Miscella abgetrennte, erste Extraktionsmittel (enthaltend Ethanol, insbesondere Bioethanol) zum Trennen des ersten Extraktionsprodukts in die erstes Mischung und in die zweite Mischung verwendet wird, insbesondere als zweites Extraktionsmittel.
57. Kombiniertes Verfahren nach einer der Ausführungsformen 1 bis 56, wobei im zweiten Verfahren ein Ölfruchtproteinkonzentrat hergestellt wird, vorzugsweise ein Sojaproteinkonzentrat.
58. Kombiniertes Verfahren nach Ausführungsform 57, wobei das erste Extraktionsprodukt in Schritt (iii) mit einem zweiten Extraktionsmittel extrahiert wird, um es in die erste Mischung und die zweite Mischung zu trennen.
59. Kombiniertes Verfahren nach Ausführungsform 58, wobei das Extrahieren in Schritt (iii) bei erhöhter Temperatur durchgeführt wird, vorzugsweise bei 25°C oder darüber, bevorzugter bei 30°C oder darüber, noch bevorzugter bei 35 bis 50°C.
60. Kombiniertes Verfahren nach Ausführungsform 59, wobei die für das Extrahieren in Schritt (iii) bei erhöhter Temperatur benötigte thermische Energie zumindest zum Teil im kombinierten Verfahren gewonnene thermische Energie ist, bevorzugt beim Abtrennen zumindest eines Teils des Bioethanols von der fermentierten Brühe in Schritt (c) gewonnene thermische Energie, und/oder beim Kondensieren des beim Trocknen der Schlempe abgetrennten Wassers gewonnene thermische Energie.
61. Kombiniertes Verfahren nach einer der Ausführungsformen 58 bis 60, wobei das zweite Extraktionsmittel Ethanol (insbesondere Bioethanol) und Wasser enthält, wobei der Ethanolgehalt vorzugsweise im Bereich von 40 bis 90 Gew% liegt, bevorzugter von 55 bis 90 Gew%, insbesondere von 60 bis 90 Gew%, bezogen auf das Gewicht des zweiten Extraktionsmittels.
62. Kombiniertes Verfahren nach Ausführungsform 61, wobei das im zweiten Extraktionsmittel enthaltene Ethanol im ersten Verfahren hergestelltes Bioethanol ist.
63. Kombiniertes Verfahren nach einer der Ausführungsformen 58 bis 62, wobei zumindest ein Teil des zweiten Extraktionsmittels von der zweiten Mischung abgetrennt wird, vorzugsweise durch Destillieren und/oder Strippen.
64. Kombiniertes Verfahren nach Ausführungsform 63, wobei das von der zweiten Mischung abgetrennte, zweite Extraktionsmittel einen Ethanolgehalt von 55 Gew% oder darüber aufweist, bevorzugter von 60 Gew% oder darüber, noch bevorzugter von 65 Gew% oder darüber, besonders bevorzugt von 70 Gew% oder darüber, bezogen auf das Gewicht des abgetrennten, zweiten Extraktionsmittels.
65. Kombiniertes Verfahren nach Ausführungsform 63 oder 64, wobei das von der zweiten Mischung abgetrennte, zweite Extraktionsmittel als zweites Extraktionsmittel wiederverwendet wird.
66. Kombiniertes Verfahren nach Ausführungsform 65, wobei das abgetrennte, zweite Extraktionsmittel vor der Wiederverwendung als zweites Extraktionsmittel gereinigt wird, wobei das Reinigen vorzugsweise ein Filtrieren umfasst.
67. Kombiniertes Verfahren nach einer der Ausführungsformen 63 bis 66, wobei das abgetrennte, zweite Extraktionsmittel Ethanol (insbesondere Bioethanol) enthält und in Schritt (c) des ersten Verfahrens verwendet wird, vorzugsweise wobei das abgetrennte, zweite Extraktionsmittel der in Schritt (b) des ersten Verfahrens erhaltenen, fermentierten Brühe zugegeben wird, bevorzugt nach einem zumindest teilweisen Abtrennen von Wasser und/oder einer Schlempe von der fermentierten Brühe, insbesondere nach einem teilweisen Destillieren der fermentierten Brühe.
68. Kombiniertes Verfahren nach einer der Ausführungsformen 57 bis 67, wobei in Schritt (iv) zumindest ein Teil des Ölfruchtproteinkonzentrats von der ersten Mischung, insbesondere vom zweiten Extraktionsmittel, abgetrennt wird, vorzugsweise mittels Verdampfens.
69. Kombiniertes Verfahren nach Ausführungsform 68, wobei für das Abtrennen zumindest eines Teils des Ölfruchtproteinkonzentrats von der ersten Mischung, insbesondere vom zweiten Extraktionsmittel, insbesondere mittels Verdampfen, benötigte thermische Energie zumindest zum Teil im kombinierten Verfahren gewonnene thermische Energie ist, bevorzugt beim Abtrennen zumindest eines Teils des Bioethanols von der fermentierten Brühe in Schritt (c) gewonnene thermische Energie, und/oder beim Kondensieren des beim Trocknen der Schlempe abgetrennten Wassers gewonnene thermische Energie.
70. Kombiniertes Verfahren nach einer der Ausführungsformen 57 bis 69, wobei das in Schritt (iv) abgetrennte Ölfruchtproteinkonzentrat getrocknet wird.
71. Kombiniertes Verfahren nach einer der Ausführungsformen 57 bis 70, wobei in Schritt (iv) zumindest ein Teil des Ölfruchtnebenprodukts enthaltend die Ölfruchtmelasse von der zweiten Mischung abgetrennt wird.
72. Kombiniertes Verfahren nach Ausführungsform 71, wobei das Abtrennen zumindest eines Teils des Ölfruchtnebenprodukts von der zweiten Mischung ein Strippen und/oder ein Dekantieren umfasst.
73. Kombiniertes Verfahren nach einer der Ausführungsformen 57 bis 72, wobei in Schritt (iv) zumindest ein Teil des zweiten Extraktionsmittels von der zweiten Mischung abgetrennt wird, vorzugsweise wobei das Abtrennen ein Destillieren und/oder Strippen umfasst.
74. Kombiniertes Verfahren nach Ausführungsform 73, wobei zumindest ein Teil der zweiten Mischung nach dem Abtrennen zumindest eines Teils des zweiten Extraktionsmittels im ersten Verfahren verwendet wird, und dieser Teil der zweiten Mischung in der in Schritt (a) bereitgestellten Maische enthalten ist.
75. Kombiniertes Verfahren nach einer der Ausführungsformen 71 bis 74, wobei das von der zweiten Mischung abgetrennte Ölfruchtnebenprodukt und/oder die zweite Mischung vor der Verwendung im ersten Verfahren eingedampft wird.
76. Kombiniertes Verfahren nach einer der Ausführungsformen 71 bis 75, wobei das im ersten Verfahren verwendete Ölfruchtnebenprodukt und/oder die im ersten Verfahren verwendete zweite Mischung ein Trockensubstanzgewicht von 10 bis 80 Gew% aufweist, bevorzugter von 15 bis 50 Gew%, noch bevorzugter von 20 bis 40 Gew%, besonders bevorzugt von 25 bis 35 Gew%, bezogen auf das Gesamtgewicht des Ölfruchtnebenprodukts und/oder der zweiten Mischung.
77. Kombiniertes Verfahren nach einer der Ausführungsformen 1 bis 56, wobei im zweiten Verfahren ein Ölfruchtproteinisolat hergestellt wird.
78. Kombiniertes Verfahren nach Ausführungsform 77, wobei zwischen den Schritten (ii) und (iii) zumindest ein Teil des im ersten Extraktionsprodukt enthaltenen ersten Extraktionsmittels vom ersten Extraktionsprodukt abgetrennt wird, vorzugsweise mittels Verdampfen; vorzugsweise wobei für das Abtrennen, insbesondere mittels Verdampfen, benötigte thermische Energie zumindest zum Teil im kombinierten Verfahren gewonnene thermische Energie ist, bevorzugt beim Abtrennen zumindest eines Teils des Bioethanols von der fermentierten Brühe in Schritt (c) gewonnene thermische Energie, und/oder beim Kondensieren des beim Trocknen der Schlempe abgetrennten Wassers gewonnene thermische Energie.
79. Kombiniertes Verfahren nach Ausführungsform 78, wobei in Schritt (iii) Wasser zum ersten Extraktionsprodukt zugegeben wird, und ein pH-Wert des ersten Extraktionsprodukts im Wesentlichen auf den isoelektrischen Punkt des Ölfruchtproteinprodukts eingestellt wird, um zumindest einen Teil des Ölfruchtproteinisolats auszufällen und somit das erste Extraktionsprodukt in die erste (ausgefällte) Mischung und in die zweite Mischung zu trennen.
80. Kombiniertes Verfahren nach Ausführungsform 78 oder 79, wobei das Abtrennen zumindest eines Teils des Ölfruchtproteinisolats von der ersten Mischung in Schritt (iv) ein Entwässern des Ölfruchtproteinisolats umfasst, insbesondere ein Verdampfen von Wasser, ein Zentrifugieren, ein Dekantieren und/oder ein Sedimentieren; wobei das abgetrennte Ölfruchtproteinisolat vorzugsweise getrocknet wird.
81. Kombiniertes Verfahren nach einer der Ausführungsformen 77 bis 80, wobei in Schritt (iv) des zweiten Verfahrens zumindest ein Teil des Ölfruchtnebenprodukts enthaltend den Ölfruchtproteinliquor von der zweiten Mischung abgetrennt wird, wobei das Abtrennen ein Entwässern des Ölfruchtnebenprodukts umfasst.
82. Kombiniertes Verfahren nach Ausführungsform 81, wobei das Abtrennen zumindest eines Teils des Ölfruchtnebenprodukts enthaltend den Ölfruchtproteinliquor von der zweiten Mischung ein Verdampfen von Wasser, ein Zentrifugieren, ein Dekantieren und/oder ein Sedimentieren umfasst.
83. Kombiniertes Verfahren nach einer der Ausführungsformen 77 bis 82, wobei zumindest ein Teil der zweiten Mischung und/oder zumindest ein Teil des von der zweiten Mischung abgetrennten Ölfruchtnebenprodukts im ersten Verfahren verwendet wird und in der in Schritt (a) bereitgestellten Maische enthalten ist.
84. Futtermittel, erhältlich durch das kombinierte Verfahren nach einer der Ausführungsformen 1 bis 83, und enthaltend Schalen von Ölfrüchten, vorzugsweise zerkleinerte Schalen von Ölfrüchten, insbesondere gemahlene Schalen von Ölfrüchten.
85. Futtermittel, erhältlich durch das kombinierte Verfahren nach einer der Ausführungsformen 1 bis 83 und enthaltend das Ölfruchtnebenprodukt, insbesondere die Ölfruchtmelasse und/oder die Ölfruchtproteinliquor.
86. Futtermittel nach Ausführungsform 85, weiters enthaltend Schalen von Ölfrüchten, bevorzugter zerkleinerte Schalen von Ölfrüchten, insbesondere gemahlene Schalen von Ölfrüchten.
87. Kombinierte Anlage zur Herstellung von Bioethanol und einem Ölfruchtproteinprodukt mit dem kombinierten Verfahren nach einer der Ausführungsformen 1 bis 83, aufweisend
   *eine erste Anlage zur Herstellung des Bioethanols, aufweisend*
      einen Fermenter zum Fermentieren der Maische, und
      eine erste Trenneinheit zum Abtrennen zumindest eines Teils des Bioethanols von der fermentierten Brühe; und
   *eine zweite Anlage zur Herstellung des Ölfruchtproteinprodukts, aufweisend*
      eine erste Extraktionsvorrichtung zum Extrahieren des Ölfruchtausgangsprodukts mit dem ersten Extraktionsmittel,
      eine zweite Trenneinheit zum Trennen des ersten Extraktionsprodukts in die erste Mischung und die zweite Mischung,
      eine dritte Trenneinheit zum Abtrennen zumindest eines Teils des Ölfruchtproteinprodukts von der ersten Mischung, und
      gegebenenfalls eine vierte Trenneinheit zum Abtrennen zumindest eines Teils des Ölfruchtnebenprodukts von der zweiten Mischung.
88. Kombinierte Anlage zur Herstellung von Bioethanol und einem Ölfruchtproteinprodukt mit dem kombinierten Verfahren nach einer der Ausführungsformen 1 bis 83, aufweisend
   *eine erste Anlage zur Herstellung des Bioethanols, aufweisend*
      einen Fermenter zum Fermentieren der Maische, und
      eine erste Trenneinheit zum Abtrennen zumindest eines Teils des Bioethanols von der fermentierten Brühe; und
   *eine zweite Anlage zur Herstellung des Ölfruchtproteinprodukts, aufweisend*
      eine erste Extraktionsvorrichtung zum Extrahieren des Ölfruchtausgangsprodukts mit dem ersten Extraktionsmittel,
      eine zweite Trenneinheit zum Trennen des ersten Extraktionsprodukts in die erste Mischung und die zweite Mischung,
      eine dritte Trenneinheit zum Abtrennen zumindest eines Teils des Ölfruchtproteinprodukts von der ersten Mischung,
      gegebenenfalls eine vierte Trenneinheit zum Abtrennen zumindest eines Teils des Ölfruchtnebenprodukts von der zweiten Mischung;
   wobei die zweite Anlage weiters eine fünfte Trenneinheit zum Abtrennen zumindest eines Teils des in der ersten Miscella enthaltenen, ersten Extraktionsmittels aufweist, vorzugsweise wobei die erste Extraktionsvorrichtung und die fünfte Trenneinheit mit der ersten Trenneinheit verbunden sind.
89. Kombinierte Anlage zur Herstellung von Bioethanol und einem Ölfruchtproteinprodukt mit dem kombinierten Verfahren nach einer der Ausführungsformen 1 bis 83, aufweisend
   *eine erste Anlage zur Herstellung des Bioethanols, aufweisend*
      einen Fermenter zum Fermentieren der Maische, und
      eine erste Trenneinheit zum Abtrennen zumindest eines Teils des Bioethanols von der fermentierten Brühe; und
   *eine zweite Anlage zur Herstellung des Ölfruchtproteinprodukts, aufweisend*
      eine erste Extraktionsvorrichtung zum Extrahieren des Ölfruchtausgangsprodukts mit dem ersten Extraktionsmittel,
      eine zweite Trenneinheit zum Trennen des ersten Extraktionsprodukts in die erste Mischung und die zweite Mischung,
      eine dritte Trenneinheit zum Abtrennen zumindest eines Teils des Ölfruchtproteinprodukts von der ersten Mischung, und
      gegebenenfalls eine vierte Trenneinheit zum Abtrennen zumindest eines Teils des Ölfruchtnebenprodukts von der zweiten Mischung;
   wobei die erste Anlage weiters eine Zerkleinerungseinheit zur Zerkleinerung des kohlenhydrathaltigen Ausgangsmaterials und von Schalen von Ölfrüchten aufweist, und
   wobei die zweite Anlage weiters eine Aufbereitungseinheit zum Aufbereiten des bereitgestellten Ölfruchtausgangsprodukts aufweist, wobei die Aufbereitungseinheit eine Schäleinheit aufweist, vorzugsweise wobei die Schäleinheit der Aufbereitungseinheit der zweiten Anlage mit der Zerkleinerungseinheit der ersten Anlage verbunden ist.
90. Kombinierte Anlage nach einer der Ausführungsformen 87 bis 89, wobei die erste Trenneinheit eine Destillationsapparatur aufweist; vorzugsweise wobei die erste Trenneinheit weiters ein Molekularsieb, eine Membran und/oder ein Adsorbens aufweist, besonders bevorzugt ein Molekularsieb; vorzugsweise wobei das Molekularsieb, die Membran und/oder das Adsorbens der Destillationsapparatur nachgeschaltet ist.
91. Kombinierte Anlage nach Ausführungsform 90, wobei ein Abschnitt der ersten Trenneinheit, die das Molekularsieb aufweist, mit dem Fermenter derart verbunden ist, dass Kohlenstoffdioxid vom Fermenter zu diesem Abschnitt der ersten Trenneinheit geführt werden kann; und/oder wobei der Abschnitt der ersten Trenneinheit, die das Molekularsieb aufweist, mit der Destillationsapparatur der ersten Trenneinheit derart verbunden ist, dass ein zur Reinigung des Molekularsiebs verwendetes, ethanolhaltiges Reinigungsmittel von dem Abschnitt der ersten Trenneinheit, die das Molekularsieb aufweist, zur Destillationsapparatur geführt werden kann; und/oder wobei der Abschnitt der ersten Trenneinheit, die das Molekularsieb aufweist, mit der zweiten Trenneinheit verbunden ist, insbesondere mit einer zweiten Extraktionsvorrichtung der zweiten Trenneinheit.
92. Kombinierte Anlage nach einer der Ausführungsformen 87 bis 91, wobei die erste Anlage weiters eine Maischapparatur zum Maischen des kohlenhydrathaltigen Ausgangsmaterials und des Ölfruchtnebenprodukts aufweist, wobei die Maischapparatur vorzugsweise mit der vierten Trenneinheit verbunden ist.
93. Kombinierte Anlage nach einer der Ausführungsformen 87 bis 92, wobei die erste Anlage weiters eine Zerkleinerungseinheit zur Zerkleinerung des kohlenhydrathaltigen Ausgangsmaterials, und gegebenenfalls von Schalen von Ölfrüchten, aufweist.
94. Kombinierte Anlage nach einer der Ausführungsformen 87 bis 93, wobei die erste Anlage weiters einen Trockner, insbesondere einen Futtermitteltrockner, zum Trocknen der Schlempe aufweist; vorzugsweise wobei der Trockner, insbesondere Futtermitteltrockner, der ersten Anlage mit der Zerkleinerungseinheit der ersten Anlage verbunden ist.
95. Kombinierte Anlage nach einer der Ausführungsformen 87 bis 94, wobei die zweite Trenneinheit eine zweite Extraktionsvorrichtung aufweist, oder wobei die zweite Trenneinheit einen Trockner und eine Fällungsapparatur aufweist.
96. Kombinierte Anlage nach einer der Ausführungsformen 87 bis 95, wobei die dritte Trenneinheit einen Trockner aufweist.
97. Kombinierte Anlage nach einer der Ausführungsformen 87 bis 96, wobei die vierte Trenneinheit einen Stripper, eine Destillationsapparatur, eine Zentrifuge, einen Dekanter, einen Sedimenter und/oder einen Verdampfer aufweist.
98. Kombinierte Anlage nach einer der Ausführungsformen 87 bis 97, wobei die zweite Anlage weiters eine fünfte Trenneinheit zum Abtrennen zumindest eines Teils des in der ersten Miscella enthaltenen, ersten Extraktionsmittels und/oder zum Abtrennen zumindest eines Teils des in der ersten Miscella enthaltenen Öls und/oder Fetts aufweist; vorzugsweise wobei die fünfte Trenneinheit eine Destillationsapparatur und/oder einen Stripper aufweist.
99. Kombinierte Anlage nach einer der Ausführungsformen 87 bis 98, wobei die zweite Anlage eine Aufbereitungseinheit zum Aufbereiten des bereitgestellten Ölfruchtausgangsprodukts aufweist, vorzugsweise wobei die Aufbereitungseinheit eine Schäleinheit und/oder eine Zerkleinerungseinheit aufweist.
100. Kombinierte Anlage nach Ausführungsform 99, wobei die Aufbereitungseinheit, insbesondere die Schäleinheit, der zweiten Anlage mit der Zerkleinerungseinheit der ersten Anlage verbunden ist; und/oder wobei die Aufbereitungseinheit, insbesondere die Schäleinheit, der zweiten Anlage mit dem Trockner, insbesondere Futtermitteltrockner, der ersten Anlage verbunden ist.
101. Kombinierte Anlage nach einer der Ausführungsformen 87 bis 100, wobei die Aufbereitungseinheit der zweiten Anlage, die erste Extraktionsvorrichtung, die zweite Trenneinheit, die dritte Trenneinheit, die vierte Trenneinheit und/oder die fünfte Trenneinheit verbunden ist/sind mit der ersten Trenneinheit (insbesondere mit einem Abschnitt der ersten Trenneinheit, die das Molekularsieb aufweist) und/oder mit dem Trockner (insbesondere Futtermitteltrockner) der ersten Anlage, insbesondere mit einem zum Transport von thermischer Energie geeigneten Verbindungselement, um in der ersten Anlage gewonnene thermische Energie im zweiten Verfahren zu verwenden.
102. Kombinierte Anlage nach einer der Ausführungsformen 87 bis 101, weiters aufweisend einen Lagerbehälter zur Lagerung von abgetrenntem ersten Extraktionsmittel, abgetrenntem zweiten Extraktionsmittel und/oder abgetrenntem Reinigungsmittel, jeweils enthaltend Ethanol, vorzugsweise Bioethanol, wobei der Lagerbehälter vorzugsweise mit der ersten Trenneinheit (insbesondere mit der Destillationsapparatur der ersten Trenneinheit) verbunden ist; und wobei der Lagerbehälter vorzugsweise weiters mit der zweiten Trenneinheit, mit der dritten Trenneinheit, mit der vierten Trenneinheit, mit der fünften Trenneinheit und/oder mit dem Abschnitt der ersten Trenneinheit, die das Molekularsieb aufweist, verbunden ist.
103. Kombinierte Anlage nach einer der Ausführungsformen 87 bis 102, weiters aufweisend eine Abwasserbehandlungseinheit, wobei die Abwasserbehandlungseinheit vorzugsweise mit der ersten Trenneinheit und/oder mit dem Trockner (insbesondere Futtermitteltrockner) der ersten Anlage verbunden ist.
104. Kombinierte Anlage nach einer der Ausführungsformen 87 bis 103, wobei die erste Anlage, insbesondere die erste Trenneinheit, einen ersten Sammelbehälter zum Sammeln des im ersten Verfahren hergestellten Bioethanols aufweist; und/oder wobei die zweite Anlage, insbesondere die dritte Trenneinheit, einen zweiten Sammelbehälter zum Sammeln des im zweiten Verfahren hergestellten Ölfruchtproteinprodukts aufweist.
105. Kombinierte Anlage nach einer der Ausführungsformen 87 bis 104, wobei die erste Extraktionsvorrichtung mit der ersten Trenneinheit, insbesondere mit dem ersten Sammelbehälter, verbunden ist.
106. Kombinierte Anlage nach einer der Ausführungsformen 87 bis 105, wobei die zweite Anlage zwei zweite Trenneinheiten, zwei dritte Trenneinheiten, gegebenenfalls zwei vierte Trenneinheiten und gegebenenfalls zwei zweite Sammelbehälter aufweist, um Ölfruchtproteinisolat und Ölfruchtproteinkonzentrat gleichzeitig und/oder hintereinander herzustellen.
107. Ölfruchtproteinprodukt, erhältlich nach einem kombinierten Verfahren nach einer der Ausführungsformen 1 bis 83, insbesondere ein Ölfruchtproteinkonzentrat, ein Ölfruchtproteinisolat, ein Ölfruchtöl-Produkt, ein Ölfrucht-Lecithin-Produkt oder ein Ölfrucht-Faserprodukt erhältlich nach einem kombinierten Verfahren nach einer der Ausführungsformen 1 bis 83.
108. Ölfruchtproteinprodukt nach Ausführungsform 106, wobei das Produkt ein Sojaproteinkonzentrat (SPC), ein Sojaproteinisolat (SPI), ein Sojaöl-Produkt, ein Soja-Lecithin-Produkt oder ein Soja-Faserprodukt ist.

### Figuren

Die Erfindung wird weiters durch die in den folgenden Figuren dargestellten, bevorzugten Ausführungsformen weiter beschrieben, auf welche sie jedoch nicht eingeschränkt ist.
Fig. 1 zeigt ein schematisches Fließdiagramm eines kombinierten Verfahrens, wobei im ersten Verfahren Bioethanol und im zweiten Verfahren ein Ölfruchtproteinkonzentrat hergestellt wird.
Fig. 2 zeigt ein schematisches Fließdiagramm eines kombinierten Verfahrens, wobei im ersten Verfahren Bioethanol und im zweiten Verfahren ein Ölfruchtproteinisolat hergestellt wird.

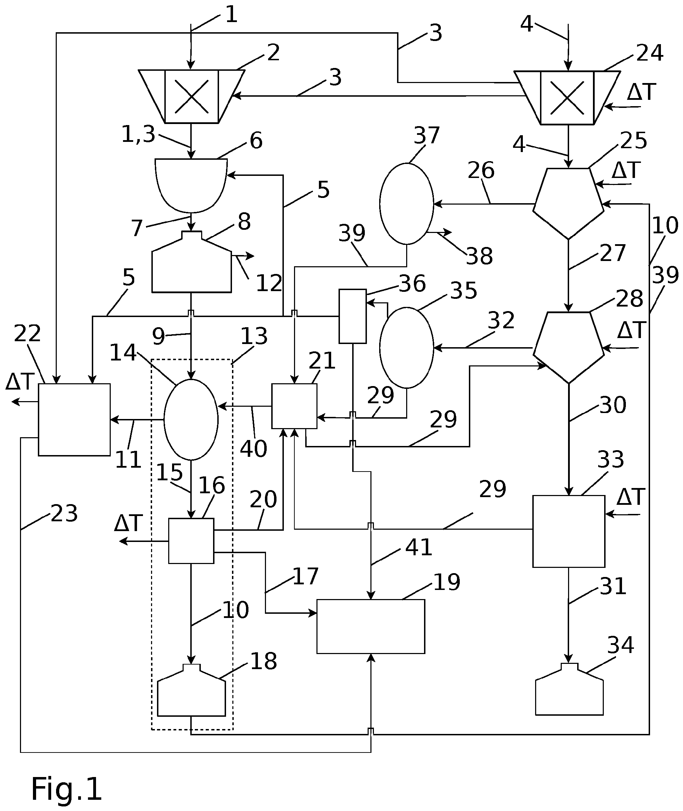

In Fig. 1 ist ein schematisches Fließdiagramm eines kombinierten Verfahrens. Wie ersichtlich ist, wird ein kohlenhydrathaltiges Ausgangsmaterial 1 in einer Zerkleinerungseinheit 2 zerkleinert. Das Zerkleinern erfolgt mittels Trockenmahlen, wobei das kohlenhydrathaltige Ausgangsmaterial 1 gemeinsam mit Schalen 3 gemischt wird, wobei die Schalen 3 zumindest ein Teil von im zweiten Verfahren von Ölfrüchten abgetrennten Schalen sind, um ein Ölfruchtausgangsprodukt 4 zu erhalten. Anschließend werden das zerkleinerte, kohlenhydrathaltige Ausgangsmaterial 1 und die zerkleinerten Schalen 3 sowie ein im zweiten Verfahren erhaltenes Ölfruchtnebenprodukt 5, das eine Ölfruchtmelasse enthält, einer Maischapparatur 6 zugegeben, wobei das Verhältnis von Ölfruchtnebenprodukt 5 zu kohlenhydrathaltigem Ausgangsmaterial 1 bei 1:10 liegt (Gew%/Gew%). In der Maischapparatur 6 erfolgt das Maischen in Anwesenheit einer Alpha-Amylase. Die Maische 7 wird dann einem Fermenter 8 zugegeben. Durch das Fermentieren wird eine fermentierte Brühe 9 enthaltend Bioethanol 10, eine Schlempe 11 und Wasser erhalten, wobei Kohlenstoffdioxid 12 als Nebenprodukt anfällt. Die fermentierte Brühe 9 wird einer ersten Trenneinheit 13 zugeführt. Die erste Trenneinheit 13 weist eine Destillationsapparatur 14 auf, in welcher die Schlempe 11 von einer Bioethanol-Wasser-Mischung 15 abgetrennt wird. Die erhaltene Bioethanol-Wasser-Mischung 15 wird anschließend mittels eines Molekularsiebs 16 der ersten Trenneinheit 13 in Bioethanol 10 und Wasser 17 getrennt. Das Bioethanol 10 kann hierbei in gasförmigem Zustand vorliegen und anschließend kondensiert werden, um es in einem ersten Sammelbehälter 18 der ersten Trenneinheit 13 zu sammeln. Dabei kann thermische Energie ΔT gewonnen werden, die im kombinierten Verfahren verwendet werden kann. Das im ersten Sammelbehälter 18 gesammelte Bioethanol 10 weist eine Reinheit von 99,0 Gew% oder darüber auf (bezogen auf das Gewicht des Bioethanols 10). Das vom Bioethanol 10 abgetrennte Wasser 17 kann einer Abwasserbehandlungseinheit 19 zur Aufbereitung zugeführt werden. Das Molekularsieb 16 kann nach seiner Verwendung mit einem ethanolhaltigen Reinigungsmittel 20 gereinigt werden. Das im Reinigungsmittel 20 enthaltene Ethanol kann im ersten Verfahren hergestelltes Bioethanol 10 sein. Nach der Verwendung des Reinigungsmittels 20 kann dieses kann zur Lagerung einem Lagerbehälter 21 zugeführt werden. Die in der fermentierten Brühe 9 enthaltene Schlempe 11 wird einem Trockner 22, insbesondere einem Futtermitteltrockner, zugeführt, um sie zu trocknen. Dem Trockner 22 können auch im zweiten Verfahren anfallende Schalen 3 zugegeben werden, mit welchen die Schlempe 11 (und die darin bereits enthaltenen, zerkleinerten Schalen 3) getrocknet werden kann. Alternativ oder zusätzlich dazu kann dem Trockner 22 ein Teil des im zweiten Verfahren erhaltenen Ölfruchtnebenprodukts 5 zugegeben werden, mit welchem die Schlempe 11 getrocknet werden kann. Die getrocknete Schlempe 11 kann als hochwertiges Futtermittel verwendet werden. Beim Trocknen der Schlempe 11 abgetrenntes, gasförmiges Wasser 23 wird nachfolgend kondensiert, wobei thermische Energie ΔT gewonnen wird. Dieses Wasser 23 kann dann der Abwasserbehandlungseinheit 19 zur Aufbereitung zugeführt werden.

Weiters ist aus Fig. 1 ersichtlich, dass ein Ölfruchtausgangsprodukt 4 durch Aufbereiten in einer Aufbereitungseinheit 24 bereitgestellt wird. Die Aufbereitungseinheit 24 weist eine Schäleinheit und eine Zerkleinerungseinheit auf (nicht dargestellt). Das Ölfruchtausgangsprodukt 4 wird zunächst in der Schäleinheit geschält, wobei die abgetrennten Schalen 3 der Zerkleinerungseinheit 1, und gegebenenfalls dem Trockner 22, zugeführt werden. Das geschälte Ölfruchtausgangsprodukt 4 wird anschließend in der Zerkleinerungseinheit der Aufbereitungseinheit 24 zerkleinert. Das Zerkleinern kann bei erhöhter Temperatur erfolgen, wobei im kombinierten Verfahren gewonnene thermische Energie ΔT verwendet werden kann. Das geschälte Ölfruchtausgangsprodukt 4 wird dann der ersten Extraktionsvorrichtung 25 zugeführt, wo es mit aus dem ersten Sammelbehälter 18 zugeführten Bioethanol 10, der als erstes Extraktionsmittel 39 verwendet wird, extrahiert wird. Das Extrahieren kann bei erhöhter Temperatur erfolgen, wobei im kombinierten Verfahren gewonnene thermische Energie ΔT verwendet werden kann. Durch das Extrahieren in der ersten Extraktionsvorrichtung 25 werden eine erste Miscella 26 und ein erstes Extraktionsprodukt 27 erhalten. Das erste Extraktionsprodukt 27 wird nachfolgend einer zweiten Trenneinheit 28 zugeführt, die eine zweite Extraktionsvorrichtung aufweist. Durch Extrahieren mit einem zweiten Extraktionsmittel 29, das Bioethanol 10 und Wasser enthält, wird das erste Extraktionsprodukt 27 in eine erste Mischung 30 enthaltend ein Ölfruchtproteinprodukt 31, das ein Ölfruchtproteinkonzentrat ist, und in eine zweite Mischung 32 (bezeichnet auch als zweite Miscella) enthaltend das Ölfruchtnebenprodukt 5 getrennt. Das Extrahieren in der zweiten Extraktionsvorrichtung kann bei erhöhter Temperatur erfolgen, wobei im kombinierten Verfahren gewonnene thermische Energie ΔT verwendet werden kann. Das zweite Extraktionsmittel 29 kann dem Lagerbehälter 21 entnommen werden. Alternativ kann das Bioethanol 10 auch dem ersten Sammelbehälter 18 entnommen werden, und Wasser kann in einer entsprechenden Menge hinzugegeben werden. Die erste Mischung 30 wird nachfolgend einer dritten Trenneinheit 33 zugeführt, um zumindest einen Teil des Ölfruchtproteinprodukts 31 von der ersten Mischung 30 abzutrennen. Die dritte Trenneinheit 33 weist einen Trockner auf. Im Trockner wird zumindest ein Teil des zweiten Extraktionsmittels 29 verdampft, und nachfolgend wird das Ölfruchtproteinprodukt 31 noch getrocknet, um etwaiges, verbliebenes, zweites Extraktionsmittel zu entfernen. Das Verdampfen und Trocknen erfolgen bei erhöhter Temperatur, wobei im kombinierten Verfahren gewonnene thermische Energie ΔT verwendet werden kann. Abgetrenntes, zweites Extraktionsmittel 29 wird dem Lagerbehälter 21 zugeführt, während das getrocknete Ölfruchtproteinprodukt 31 in einem zweiten Sammelbehälter 34 gesammelt werden kann.

Aus Fig. 1 ist weiters ersichtlich, dass die zweite Mischung 32 enthaltend das Ölfruchtnebenprodukt 5 einer vierten Trenneinheit 35 zugeführt wird, um zumindest einen Teil des Ölfruchtnebenprodukts 5 von der zweiten Mischung 32 abzutrennen. Für diese Zwecke weist die vierte Trenneinheit 35 einen Stripper auf. Mittels des Strippers kann zumindest ein Teil des zweiten Extraktionsmittel 29 von der zweiten Mischung 32 abgetrennt und nachfolgend dem Lagerbehälter 21 zur Wiederverwendung im kombinierten Verfahren zugeführt werden. Das Ölfruchtnebenprodukt 5 wird dann der Maischapparatur 6 zugeführt. Alternativ könnte es dem zerkleinerten kohlenhydrathaltigen Ausgangsmaterial 1 und den zerkleinerten Schalen 3 auch schon vor der Zugabe zur Maischapparatur 6 beigemengt werden. Vorgehend kann das Ölfruchtnebenprodukt 5 mittels eines Verdampfers 36 eingedampft werden, um den Wassergehalt zu verringern. Das abgetrennte Wasser 41 kann der Abwasserbehandlungseinheit 19 zugeführt werden.

Weiters ist Fig. 1 entnehmbar, dass die erste Miscella 26 aus der ersten Extraktionsvorrichtung 25 einer fünften Trenneinheit 37 zugeführt wird. Die fünfte Trenneinheit 37 weist einen Stripper auf, in welchem zumindest ein Teil eines Öls und/oder Fetts 38 und zumindest ein Teil des in der ersten Miscella 26 enthaltenen, ersten Extraktionsmittels 39 von der ersten Miscella 26 abgetrennt werden. Das abgetrennte Öl und/oder Fett 38 kann rückgewonnen werden und für andere Anwendungsgebiete zum Einsatz kommen. Das abgetrennte, erste Extraktionsmittel 39 wird dem Lagerbehälter 21 zugeführt. Alternativ oder zusätzlich könnte das abgetrennte, erste Extraktionsmittel 39 auch der zweiten Extraktionsvorrichtung der zweiten Trenneinheit 28 zur Verwendung als zweites Extraktionsmittel 29 zugeführt werden, wobei die Konzentration gegebenenfalls mit Wasser angepasst werden kann.

Fig. 1 zeigt auch, dass der Lagerbehälter 21 mit der Destillationsapparatur 14 verbunden ist. Dies ermöglicht es, je nach Bedarf, Verfügbarkeit und/oder Kapazität eine im Lagerbehälter 21 gelagerte, (bio-)ethanolhaltige Mischung 40 der Destillationsapparatur 14 zuzuführen. Die Mischung 40 kann dann zusammen mit der fermentierten Brühe 9 in Schritt (c) des ersten Verfahrens aufbereitet werden. Vorzugsweise wird die Mischung 40 bzw. ein Teil davon erst dann der Destillationsapparatur 14 zugeführt, nachdem die fermentierten Brühe 9 bereits teilweise destilliert wurde, und dementsprechend ein in der fermentierten Brühe 9 enthaltenes Wasser und/oder die Schlempe 11 bereits zumindest teilweise von der fermentierten Brühe 9 abgetrennt wurde(n). Zumindest ein Teil des in der Mischung 40 enthaltenen Ethanols bzw. Bioethanols kann gemeinsam mit zumindest einem Teil des in der (vorzugsweise teilweise destillierten) fermentierten Brühe 9 enthaltenen Bioethanols 10 von der fermentierten Brühe 9 abgetrennt werden.

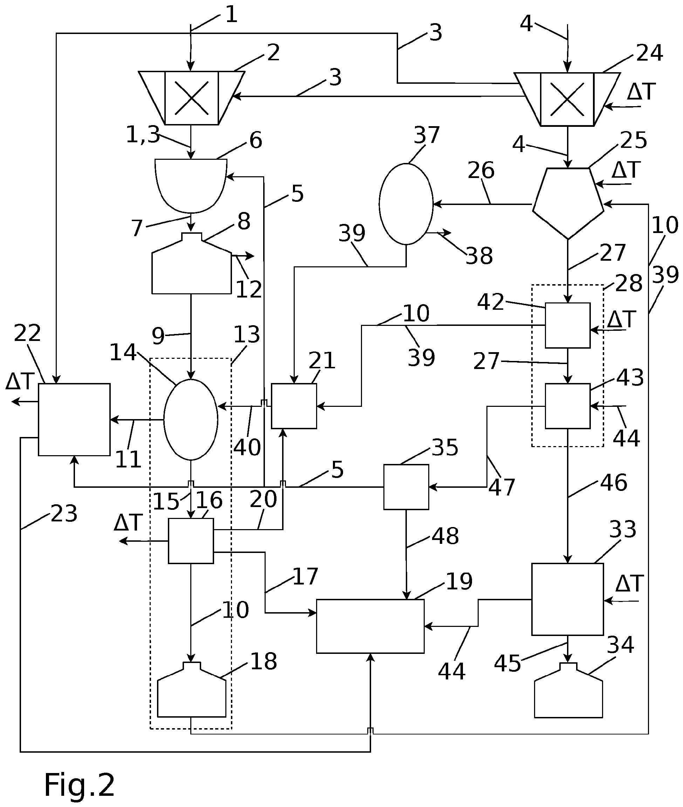

Fig. 2 zeigt ein weiteres schematisches Fließdiagramm eines kombinierten Verfahrens. Das kombinierte Verfahren der Fig. 2 entspricht großteils jenem der Fig. 1; die nachfolgende Diskussion beschränkt sich auf die Unterschiede. Das zweite Verfahren unterscheidet sich insbesondere dahingehend von dem in Fig. 1 gezeigten Verfahren, dass die zweite Trenneinheit 28 einen Trockner 42 und eine Fällungsapparatur 43 aufweist. Das durch Extrahieren in der ersten Extraktionsvorrichtung 25 erhaltene erste Extraktionsprodukt 27 wird dem Trockner 42 zugeführt. Dadurch wird zumindest ein Teil des im ersten Extraktionsprodukt 27 enthaltenen, ersten Extraktionsmittels 39 (wobei Bioethanol 10 aus dem ersten Sammelbehälter 18 als erstes Extraktionsmittel 39 verwendet wurde), vom ersten Extraktionsprodukt 27 abgetrennt. Dies erfolgt durch Verdampfen des Bioethanols 10 bei erhöhter Temperatur, wobei im kombinierten Verfahren gewonnene thermische Energie ΔT verwendet werden kann. Das verdampfte Bioethanol 10 kann nachfolgend kondensiert werden; die hierbei gewonnene thermische Energie kann im kombinierten Verfahren verwendet werden. Das Bioethanol 10 kann nachfolgend dem Lagerbehälter 21 zugeführt werden, insbesondere, wenn es verunreinigt ist. Das nach dem Verdampfen zumindest eines Teils des Bioethanols 10 verbleibende erste Extraktionsprodukt 27 wird nachfolgend in die Fällungsapparatur 43 überführt, wobei dieser auch Wasser 44 zugegeben wird. Dieses Wasser 44 kann beispielsweise der Abwasserbehandlungseinheit 19 entnommen werden. Ein pH-Wert des ersten Extraktionsprodukts 27 wird im Wesentlichen auf den isoelektrischen Punkt eines herzustellenden Ölfruchtproteinprodukts 45, das ein Ölfruchtproteinisolat ist, eingestellt, um zumindest einen Teil des Ölfruchtproteinprodukts 45 auszufällen. Dadurch kann das erste Extraktionsprodukt 27 in die erste (ausgefällte) Mischung 46 und in die zweite Mischung 47 getrennt werden. Die erste Mischung 46 enthält das Ölfruchtproteinprodukt 45 sowie gegebenenfalls eine geringe Menge des Wassers 44. Die zweite Mischung 47 enthält ein Ölfruchtnebenprodukt 5, das einen Ölfruchtproteinliquor enthält, sowie den Großteil des Wassers 44.

Wie weiters aus Fig. 2 ersichtlich ist, wird die erste Mischung 46 anschließend der dritten Trenneinheit 33 zugeführt, um zumindest einen Teil des Ölfruchtproteinprodukts 45 von der ersten Mischung 46 abzutrennen. Für diese Zwecke weist die dritte Trenneinheit 33 einen Trockner auf, in welchem das Ölfruchtproteinprodukt 45 getrocknet wird. Dies erfolgt bei erhöhter Temperatur, wobei im kombinierten Verfahren gewonnene thermische Energie ΔT verwendet werden kann. Beim Trocknen kann zumindest ein Teil des Wassers 44 abgetrennt und der Abwasserbehandlungseinheit 19 zugeführt werden. Das getrocknete Ölfruchtproteinprodukt 45 kann im zweiten Sammelbehälter 34 gesammelt werden. Die zweite Mischung 47 wird der vierten Trenneinheit 35 zugeführt, um zumindest einen Teil des Ölfruchtnebenprodukts 5 von der zweiten Mischung 47 abzutrennen. Für diese Zwecke weist die vierte Trenneinheit 35 einen Verdampfer auf. Hierdurch kann der Wassergehalt des Ölfruchtnebenprodukts 5 verringert werden. Vom Ölfruchtnebenprodukt 5 abgetrenntes Wasser 48 kann der Abwasserbehandlungseinheit 19 zur Aufbereitung zugeführt werden.

## Patentansprüche

1. Kombiniertes Verfahren, umfassend ein erstes Verfahren zur Herstellung von Bioethanol (10) und ein zweites Verfahren zur Herstellung eines Ölfruchtproteinprodukts (31, 45),
*das erste Verfahren umfassend die Schritte*
(a) Bereitstellen einer Maische (7), wobei die Maische (7) ein kohlenhydrathaltiges Ausgangsmaterial (1), insbesondere ein Getreide, enthält,
(b) Fermentieren der Maische (7), um eine fermentierte Brühe (9) enthaltend Bioethanol (10) zu erhalten, und
(c) Abtrennen zumindest eines Teils des Bioethanols (10) von der fermentierten Brühe (9); und
*das zweite Verfahren umfassend die Schritte*
(i) Bereitstellen eines Ölfruchtausgangsprodukts (4),
(ii) Extrahieren des Ölfruchtausgangsprodukts (4) mit einem ersten Extraktionsmittel (39), um eine erste Miscella (26) und ein erstes Extraktionsprodukt (27) zu erhalten,
(iii) Trennen des ersten Extraktionsprodukts (27) in eine erste Mischung (30, 46) enthaltend das Ölfruchtproteinprodukt (31, 45) und in eine zweite Mischung (32, 47) enthaltend das Ölfruchtnebenprodukt (5),
(iv) Abtrennen zumindest eines Teils des Ölfruchtproteinprodukts (31, 45) von der ersten Mischung (30, 46), und gegebenenfalls Abtrennen zumindest eines Teils des Ölfruchtnebenprodukts (5) von der zweiten Mischung (32, 47);
wobei die in Schritt (a) des ersten Verfahrens bereitgestellte Maische (7) weiters zumindest einen Teil des im zweiten Verfahren erhaltenen Ölfruchtnebenprodukts (5) enthält oder zumindest ein Teil des im zweiten Verfahren erhaltenen Ölfruchtnebenprodukts wahrend Schritt (b) zugesetzt wird,
wobei das erste Extraktionsmittel (39) im ersten Verfahren hergestelltes Bioethanol (10) enthält,
wobei die erste Miscella (26) zumindest einen Teil des ersten Extraktionsmittels (39) enthält, und
wobei zumindest ein Teil des in der ersten Miscella (26) enthaltenen, ersten Extraktionsmittels (39) von der ersten Miscella (26) abgetrennt und in Schritt (c) des ersten Verfahrens verwendet wird.

2. Kombiniertes Verfahren nach Anspruch 1, wobei die in Schritt (a) bereitgestellte Maische (7) das Ölfruchtnebenprodukt (5) in einer Menge von 1 bis 20 Gew% enthält, bezogen auf das Gewicht des kohlenhydrathaltigen Ausgangsmaterials (1).

3. Kombiniertes Verfahren nach Anspruch 1 oder 2, wobei das Bereitstellen des Ölfruchtausgangsprodukts (4) in Schritt (i) ein Abtrennen von Schalen von Ölfrüchten und/oder ein Zerkleinern der Ölfrüchte umfasst, wobei die Ölfrüchte vorzugsweise Sojabohnen, Sonnenblumenkörner und/oder Rapskörner sind.

4. Kombiniertes Verfahren nach einem der Ansprüche 1 bis 3, wobei das kohlenhydrathaltige Ausgangsmaterial Mais und/oder Weizen ist.

5. Kombiniertes Verfahren nach einem der Ansprüche 1 bis 4, wobei das abgetrennte, erste Extraktionsmittel (39) der in Schritt (b) des ersten Verfahrens erhaltenen, fermentierten Brühe (9) zugegeben wird, insbesondere nach einem zumindest teilweisen Abtrennen von Wasser und/oder einer Schlempe (11) von der fermentierten Brühe (9).

6. Kombiniertes Verfahren nach einem der Ansprüche 1 bis 5, wobei das Bereitstellen des Ölfruchtausgangsprodukts (4) in Schritt (i) des zweiten Verfahrens ein Abtrennen von Schalen (3) von Ölfrüchten umfasst, und wobei die in Schritt (a) des ersten Verfahrens bereitgestellte Maische (7) weiters zerkleinerte Schalen (3) von Ölfrüchten enthält, wobei die Schalen (3) zumindest ein Teil der in Schritt (i) des zweiten Verfahrens von den Ölfrüchten abgetrennten Schalen (3) sind.

7. Kombiniertes Verfahren nach einem der Ansprüche 1 bis 6, wobei in Schritt (c) zumindest ein Teil einer in der fermentierten Brühe (9) enthaltenen Schlempe (11) abgetrennt wird, wobei die abgetrennte Schlempe (11) nachfolgend getrocknet wird.

8. Kombiniertes Verfahren nach Anspruch 7, wobei der abgetrennten Schlempe (11) ein Teil des im zweiten Verfahren erhaltenen Ölfruchtnebenprodukts (5) zugegeben wird.

9. Futtermittel, erhältlich durch das kombinierte Verfahren nach einem der Ansprüche 1 bis 8 und enthaltend das Ölfruchtnebenprodukt (5).

10. Kombinierte Anlage zur Herstellung von Bioethanol (10) und einem Ölfruchtproteinprodukt (31, 45) mit dem kombinierten Verfahren nach einem der Ansprüche 1 bis 8, aufweisend
*eine erste Anlage zur Herstellung des Bioethanols (10), aufweisend*
einen Fermenter (8) zum Fermentieren der Maische (7), und
eine erste Trenneinheit (13) zum Abtrennen zumindest eines Teils des Bioethanols (10) von der fermentierten Brühe (9); und
*eine zweite Anlage zur Herstellung des Ölfruchtproteinprodukts (31, 45), aufweisend*
eine erste Extraktionsvorrichtung (25) zum Extrahieren des Ölfruchtausgangsprodukts (4) mit dem ersten Extraktionsmittel (39),
eine zweite Trenneinheit (28) zum Trennen des ersten Extraktionsprodukts (27) in die erste Mischung (30, 46) und die zweite Mischung (32, 47),
eine dritte Trenneinheit (33) zum Abtrennen zumindest eines Teils des Ölfruchtproteinprodukts (31, 45) von der ersten Mischung (30, 46), und
gegebenenfalls eine vierte Trenneinheit (35) zum Abtrennen zumindest eines Teils des Ölfruchtnebenprodukts (5) von der zweiten Mischung (32, 47);
wobei die zweite Anlage weiters eine fünfte Trenneinheit (37) zum Abtrennen zumindest eines Teils des in der ersten Miscella (26) enthaltenen, ersten Extraktionsmittels (39) aufweist.

11. Kombinierte Anlage nach Anspruch 10, wobei die erste Anlage weiters einen Trockner (22), insbesondere einen Futtermitteltrockner, zum Trocknen der Schlempe (11) aufweist.

12. Kombinierte Anlage nach Anspruch 10 oder 11, wobei die vierte Trenneinheit (35) mit dem Trockner (22) verbunden ist.

13. Kombinierte Anlage nach einem der Ansprüche 10 bis 12, wobei die erste Extraktionsvorrichtung (25) mit der ersten Trenneinheit (13) verbunden ist.

14. Kombinierte Anlage nach einem der Ansprüche 10 bis 13, wobei die erste Anlage weiters eine Zerkleinerungseinheit (2) zur Zerkleinerung des kohlenhydrathaltigen Ausgangsmaterials (1) und von Schalen (3) von Ölfrüchten aufweist, und wobei die zweite Anlage weiters eine Aufbereitungseinheit (24) zum Aufbereiten des bereitgestellten Ölfruchtausgangsprodukts (4) aufweist, wobei die Aufbereitungseinheit (24) eine Schäleinheit aufweist.

15. Kombinierte Anlage nach Anspruch 14, wobei die Schäleinheit der zweiten Anlage mit der Zerkleinerungseinheit (2) und/oder mit dem Trockner (22) der ersten Anlage verbunden ist.
